# EUROPEAN PATENT APPLICATION

(11) **EP 1 734 118 A1**
(43) Date of publication of application: **20.12.2006**
(21) Application number: 05012934.5
(22) Date of filing: 15.06.2005
(51) Int. Cl.: C12N 15/11, C12Q 1/68, A61K 31/713, A61K 38/17, G01N 33/68, C07K 14/47

(54) **Identification of JAK/STAT pathway modulating genes by genome wide RNAi screening**

(71) Applicant: Deutsches Krebsforschungszentrum Stiftung des öffentlichen Rechts, 69120 Heidelberg (DE); Max-Planck-Gesellschaft zur Förderung der Wissenschaften e.V., 80539 München (DE)
(72) Inventor: Boutros, Michael, Dr., 69117 Heidelberg (DE); Zeidler, Martin, 37083 Göttingen (DE); Müller, Patrick, 37073 Göttingen (DE)
(74) Representative: Weiss, Wolfgang

(57) **Abstract**

The present invention relates to a method for identifying a compound capable of modulating the activity of the JAK/STAT pathway and to the use of different JAK/STAT pathway components as a target for the modulation of the activity of the JAK/STAT pathway. Moreover, the present invention is concerned with a method for modulating the activity of the JAK/STAT pathway. Furthermore, the present invention pertains to a pharmaceutical composition and to the use of different JAK/STAT pathway components and/or effector molecules thereof for the manufacture of such composition for the diagnosis, prevention or treatment of a JAK/STAT pathway associated disorder.

## Description

The present invention relates to a method for identifying a compound capable of modulating the activity of the JAK/STAT pathway and to the use of different JAK/STAT pathway components as a target for the modulation of the activity of the JAK/STAT pathway. Moreover, the present invention is concerned with a method for modulating the activity of the JAK/STAT pathway. Furthermore, the present invention pertains to a pharmaceutical composition and to the use of different JAK/STAT pathway components and/or effector molecules thereof for the manufacture of such composition for the diagnosis, prevention or treatment of a JAK/STAT pathway associated disorder.

Signalling pathways mediating the transduction of information between cells are essential for development, cellular differentiation and homeostasis (Brivanlou, A. H. & Darnell, J. E., Jr., Science 295, 813-8. (2002)). Their dysregulation is also frequently associated with human malignancies. The JAK/STAT pathway represents one such signalling cascade whose evolutionarily conserved roles include cell proliferation and haematopoiesis (Hombria, J. C. & Brown, S., Curr Biol 12, R569-75 (2002)).

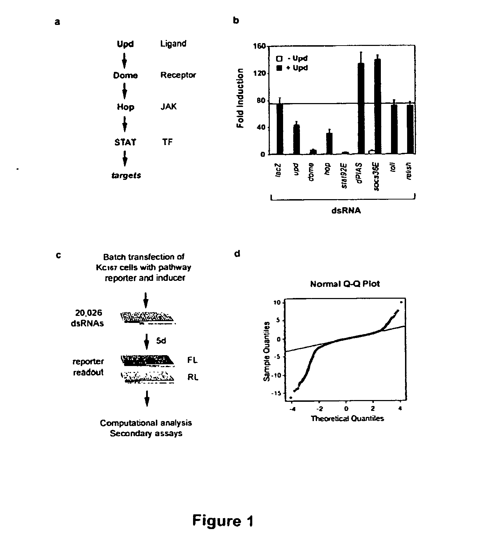

Developmental genetic screens in *Drosophila* have identified multiple JAK/STAT pathway components on the basis of their segmentation phenotype (Binari, R. & Perrimon, N., Genes Dev 8, 300-12. (1994); Harrison, D. A., McCoon, P. E., Binari, R., Gilman, M. & Perrimon, N., Genes Dev 12, 3252-63. (1998); Hou, X. S., Melnick, M. B. & Perrimon, N., Cell 84, 411-9 (1996)) and subsequent analysis of the pathway has characterised evolutionarily conserved roles during immune responses, haematopoiesis and cellular proliferation (Lagueux, M., Perrodou, E., Levashina, E. A., Capovilla, M. & Hoffmann, J. A., Proc Natl Acad Sci U S A 97, 11427-32. (2000); Boutros, M., Agaisse, H. & Perrimon, N., Dev Cell 3, 711-22. (2002); Meister, M. & Lagueux, M., Cell Microbiol 5, 573-580 (2003); Mukherjee, T., Castelli-Gair Hombría, J. & Zeidler, M. P., Oncogene in press (2005)). The JAK/STAT signalling cascade in *Drosophila* is comprised of the extracellular ligand Unpaired (Upd) (Harrison, D. A., McCoon, P. E., Binari, R., Gilman, M. & Perrimon, N., Genes Dev 12, 3252-63. (1998)), a trans-membrane receptor with homology to the IL6 receptor family termed Domeless (Dome) (Brown, S., Hu, N. & Castelli-Gair Hombria, J., Curr Biol 11, 1700-5. (2001)), a single Janus tyrosine kinase (JAK) called Hopscotch (Hop) (Binari, R. & Perrimon, N., Genes Dev 8, 300-12. (1994)) and the STAT92E transcription factor (Hou, X. S., Melnick, M. B. & Perrimon, N., Cell 84, 411-9 (1996); Yan, R., Small, S., Desplan, C., Dearolf, C. R. & Darnell, J. E., Jr., Cell 84, 421-30 (1996)) (Fig. 1a). Known regulators of JAK/STAT signalling including a family of *SOCS*-like genes (Callus, B. A. & Mathey-Prevot, B.; Oncogene 21, 4812-4821 (2002); Karsten, P., Hader, S. & Zeidler, M. P., Mech Dev 117, 343-6 (2002)), *dPIAS*/*Su(var)2-10* (Betz, A., Lampen, N., Martinek, S., Young, M. W. & Darnell, J. E., Jr., Proc Natl Acad Sci U S A 98, 9563-8 (2001)) and *STAM* (Mesilaty-Gross, S., Reich, A., Motro, B. & Wides, R., Gene 231, 173-86 (1999)) are functionally conserved and were identified based on their homology to components originally characterised in mammalian cell culture studies (Hombria, J. C. & Brown, S., Curr Biol 12, R569-75 (2002)). Although successful in identifying the pathway members Upd, Dome, Hop and STAT92E, it is probable that forward genetic approaches have missed components possibly due to non-saturating mutagenesis, genetic redundancy or phenotypic pleiotropy (Nagy, A., Perrimon, N., Sandmeyer, S. & Plasterk, R., Nat Genet 33 Suppl, 276-84 (2003)).

In order to identify novel pathway components and circumvent limitations of classical genetic screens, the inventors of the present invention have undertaken a genome-wide RNA interference (RNAi) screen, a powerful technique for the identification of new components of diverse cellular pathways (Kamath, R. S. et al., Nature 421, 231-7 (2003); Kittler, R. et al., Nature 432, 1036-40 (2004); Bems, K. et al., Nature 428, 431-7 (2004); Paddison, P. J. et al., Nature 428, 427-31 (2004); Boutros, M. et al., Science 303, 832-5 (2004)). Using this screen, a systematic genome-wide survey for genes required for JAK/STAT pathway activity could be performed. Analysis of 20,026 RNAi-induced phenotypes in cultured *Drosophila melanogaster* haemocyte-like cells identified interacting genes encoding 4 known and 84 previously uncharacterised proteins. Subsequently, cell based epistasis experiments have been used to classify these based on their interaction with known components of the signalling cascade. In addition to multiple human disease gene homologues, the inventors of the present invention have identified the tyrosine phosphatase Ptp61 F and the *Drosophila* homologue of *BRWD3,* a bromo-domain containing protein disrupted in leukaemia. Moreover, *in vivo* analysis demonstrates that disrupted *dBRWD3* and overexpressed *Ptp61F* function as suppressors of leukaemia-like blood cell tumours. This screen represents a comprehensive identification of novel loci required for JAK/STAT signalling and provides molecular insights into an important pathway relevant for human diseases.

A first aspect of the present invention, therefore, relates to a method for identifying a compound capable of modulating the activity of the JAK/STAT pathway, comprising
(a) contacting a compound with at least one target molecule selected from
   (i) nucleic acid molecules, comprising
      (i.1) a nucleotide sequence as shown in SEQ ID NOs. 88 to 265;
      (i.2) a nucleotide sequence which is complementary to a nucleotide sequence of (i.1);
      (i.3) a nucleotide sequence which has an identity of at least 65 % to a nucleotide sequence of (i.1) or (i.2); and/or
      (i.4) a nucleotide sequence which hybridizes under stringent conditions to a nucleotide sequence of (i.1), (i.2) or (i.3);
      and
   (ii) polypeptide molecules
      (ii.1) encoded by the nucleic acid molecules of (i) and/or
      (ii.2) having the sequences as shown in SEQ ID NOs. 1-87,
      and
(b) determining the degree of modulation of the at least one target molecule by the compound.

In accordance with the present invention, it is to be understood, that the term "modulating the activity of the JAK/STAT pathway", when used herein, means activating or inhibiting the activity of the JAK/STAT signalling pathway. An activation or inhibition of the activity of the JAK/STAT signalling pathway may e.g. be mediated by an activation or inhibition of at least one component of the JAK/STAT pathway, either directly or indirectly.

According to the present invention, step (a) of the method for identifying a compound capable of modulating the activity of the JAK/STAT pathway comprises contacting a compound with at least one target molecule selected from the nucleic acid molecules of (i) and the polypeptide molecules of (ii).

The nucleic acid molecules of (i) used according to the method for identifying a compound capable of modulating the activity of the JAK/STAT pathway comprise in one embodiment of the present invention a nucleotide sequence of (i.1) as show in SEQ ID NOs. 88 to 265. Preferably, the nucleic acid molecules of (i) comprise a nucleic acid sequence of (i.1) as shown in SEQ ID NOs. 88 to 174. More preferably, the nucleic acid molecules of (i) comprise a nucleic acid sequence of (i.1) as shown in SEQ ID NOs. 91, 116, 124, 133, 136, 152, 154, 155 to 174.

It is to be understood that the Drosophila gene sequences of SEQ ID Nos. 175-265 encompasse respective splice variants.

Moreover, nucleic acid molecules of (i) used according to the method for identifying a compound capable of modulating the activity of the JAK/STAT pathway comprise in another embodiment of the present invention a nucleotide sequence of (i.2) which is complementary to a nucleotide sequence of (i.1). Preferably, the nucleic acid molecules of (i) comprise a nucleic acid sequence of (i.2) which is complementary to a nucleotide sequence of (i.1) as shown in SEQ ID NOs. 88 to 174. More preferably, the nucleic acid molecules of (i) comprise a nucleic acid sequence of (i.2) which is complementary to a nucleotide sequence of (i.1) as shown in SEQ ID NOs. 91, 116, 124, 133, 136, 152, 154, 155 to 174.

In a further embodiment of the present invention, the nucleic acid molecules of (i) used according to the method for identifying a compound capable of modulating the activity of the JAK/STAT pathway comprise a nucleotide sequence of (i.3) which has an identity of at least 65, preferably at least 70, more preferably at least 75 and most preferably at least 80 % to a nucleotide sequence of (i.1) or (i.2). Within the context of the present application, the term "has an identity of at least 65, preferably at least 70, more preferably at least 75 and most preferably at least 80 %", as used herein, means that the sequence identity is at least 65, 66, 67, 6, 69, preferably at least 70, 71, 72, 73, 74, more preferably at least 75, 76, 77, 78, 79 and most preferably at least 80, 81, 82, 83, 84, 85, 86, 87, 88, 89, 90, 91, 92, 93, 94, 95, 96, 97, 98, 99 or 100 %. Preferably, the nucleic acid molecules of (i) comprise a nucleotide sequence of (i.3) which has an identity of at least 65, preferably at least 70, more preferably at least 75 and most preferably at least 80 % to a nucleotide sequence of (i.1) as shown in SEQ ID NOs. 88 to 174 or a nucleotide sequence of (i.2) which is complementary to a nucleotide sequence of (i.1) as shown in SEQ ID NOs. 88 to 174. More preferable, the nucleic acid molecules of (i) comprise a nucleotide sequence of (i.3) which has an identity of at least 65, preferably at least 70, more preferably at least 75 and most preferably at least 80 % to a nucleotide sequence of (i.1) as shown in SEQ ID NOs. 91, 116, 124, 133, 136, 152, 154, 155 to 174 or a nucleotide sequence of (i.2) which is complementary to a nucleotide sequence of (i.1) as shown in SEQ ID NOs. 91, 116, 124, 133, 136, 152, 154, 155 to 174.

Finally, the nucleic acid molecules of (i) used according to the method for identifying a compound capable of modulating the activity of the JAK/STAT pathway comprise in a further embodiment of the present invention a nucleotide sequence of (i.4) which hybridizes under stringent conditions to a nucleotide sequence of (i.1), (i.2) or (i.3). The term "hybridizes under stringent conditions" according to the present application is used as described in Sambrook et al., Molecular Cloning, A Laboratory Manual, Cold Spring Harbor, Laboratory Press (1989), 1.101-1.104. Consequently, hybridization under stringent conditions occurs when a positive hybridization signal is still detected after washing for 1 h with 1 x SSC and 0.1 % SDS at 55 °C, preferably at 62 °C and most preferably at 68 °C, in particular for 1 h in 0.2 x SSC and 0.1% SDS at 55 °C, preferably at 62 °C and most preferably at 68 °C. It is preferred that the nucleic acid molecules of (i) comprise a nucleotide sequence of (i.4) which hybridizes under stringent conditions to a nucleotide sequence of (i.1) as shown in SEQ ID NOs. 88 to 174, a nucleotide sequence of (i.2) which is complementary to a nucleotide sequence of (i.1) as shown in SEQ ID NOs. 88 to 174 or a nucleotide sequence of (i.3) which has an identity of at least 65, preferably at least 70, more preferably at least 75 and most preferably at least 80 % to a nucleotide sequence of (i.1) as shown in SEQ ID NOs. 88 to 174 or a nucleotide sequence of (i.2) which is complementary to a nucleotide sequence of (i.1) as shown in SEQ ID NOs. 88 to 174. More preferably, the nucleic acid molecules of (i) comprise a nucleotide sequence of (i.4) which hybridizes under stringent conditions to a nucleotide sequence of (i.1) as shown in SEQ ID NOs. 91, 116, 124, 133, 136, 152, 154, 155 to 174, a nucleotide sequence of (i.2) which is complementary to a nucleotide sequence of (i.1) as shown in SEQ ID NOs. 91, 116, 124, 133, 136, 152, 154, 155 to 174 or a nucleotide sequence of (i.3) which has an identity of at least 65, preferably at least 70, more preferably at least 75 and most preferably at least 80 % to a nucleotide sequence of (i.1) as shown in SEQ ID NOs. 91, 116, 124, 133, 136, 152, 154, 155 to 174 or a nucleotide sequence of (i.2) which is complementary to a nucleotide sequence of (i.1) as shown in SEQ ID NOs. 91, 116, 124, 133, 136, 152, 154, 155 to 174.

The nucleic acid molecules of (i) used according to the method for identifying a compound capable of modulating the activity of the JAK/STAT pathway may be present in single-stranded or double-stranded form and may be selected from RNA, DNA or nucleic acid analog molecules, such as sugar- and backbone-modified ribonucleic acids or deoxyribonucleic acids. It should be noted, however, that other nucleic acid analogs, such as peptide nucleic acids (PNA) or locked nucleic acids (LNA), are also suitable.

Moreover, according to the present invention, the nucleic acid molecules of (i) used according to the present invention may be non-recombinant nucleic acid molecules, recombinant nucleic acid molecules generated by recombinant methods, e.g. by known amplification procedures such as PCR, or chemically synthesized nucleic acid molecules. The nucleic acid molecules of (i) may be present in isolated, i.e. purified, form or in non-isolated form, i.e. in a cellular environment.

In a preferred embodiment of the present invention, the nucleic acid molecules of (i) used according to the present invention are present in a vector, which may be any prokaryotic or eukaryotic vector, on which the nucleic acid sequence is present preferably under control of a suitable expression signal, e.g. promoter, operator, enhancer etc. Examples for prokaryotic vectors are chromosomal vectors, such as bacteriophages, and extrachromosomal vectors, such as plasmids, wherein circular plasmid vectors are preferred. Examples for eukaryotic vectors are yeast vectors or vectors suitable for higher cells, e.g. insect cells or mammalian cells, plasmids or viruses.

The polypeptide molecules of (ii) used according to the method for identifying a compound capable of modulating the activity of the JAK/STAT pathway are encoded by the nucleic acid molecules of (i) described above and or have a sequence as shown in SED ID Nos. 1-87. According to a preferred embodiment of the present invention, the polypeptide molecules of (ii) have an amino acid sequence as shown in SEQ ID NO. 4, 29, 37, 46, 49, 65, 67 to 87.

The compound used in step (a) of the method for identifying a compound capable of modulating the activity of the JAK/STAT pathway may be selected from compounds capable of directly and/or indirectly inhibiting or activating the transcription or translation of a nucleic acid molecule of (i). Preferably, the compounds capable of directly and/or indirectly inhibiting or activating the transcription or translation of a nucleic acid molecule of (i) comprise polypeptides such as proteins, enzymes, antibodies, polypeptide inhibitors, polypeptide activators, agonist, antagonists, mimetics, low molecular weight substances, antisense molecules, RNAi molecules and ribozymes. More preferably, the compounds capable of directly and/or indirectly inhibiting or activating the transcription or translation of a nucleic acid molecule of (i) are antisense molecules directed against a nucleic acid molecule of (i) or RNAi molecules. The antisense molecules and RNAi molecules may be prepared by any method known in the art for the synthesis of nucleic acid molecules. These include techniques for chemically synthesising oligonucleotides such as solid phase phosphoramidite chemical synthesis. Altematively, said molecules may be generated by *in vitro* and *in vivo* transcription of DNA sequences.

Moreover, the compound used in step (a) of the method for identifying a compound capable of modulating the activity of the JAK/STAT pathway may also be selected from compounds capable of directly and/or indirectly inhibiting or activating a polypeptide molecule of (ii). Preferably, the compounds capable of directly and/or indirectly inhibiting or activating a polypeptide molecule of (ii) comprise polypeptides such as proteins, enzymes, antibodies, polypeptide inhibitors, polypeptide activators, agonist, antagonists, mimetics, oligopeptides, low molecular weight substances and polypeptide cofactors. More preferably, the compounds capable of directly and/or indirectly inhibiting or activating a polypeptide molecule of (ii) are antibodies or fragments thereof directed against a polypeptide molecule of (ii). Within the context of the present invention, the term "antibody", as used herein, encompasses polyclonal antibodies, monoclonal antibodies, e. g. chimeric antibodies, humanized antibodies, human antibodies or recombinant antibodies, e. g. single-chain antibodies. Further, the term "antibody fragment" encompasses common antibody fragments, e.g. proteolytic fragments such as Fab, F(ab)₂, Fab' or recombinant fragments such as scFv. The antibodies or fragments thereof may be obtained using hybridoma cell lines or recombinant DNA methods using techniques well known in the art. However, the antibodies or fragments thereof may also be isolated from phage antibody libraries using techniques described in the art.

According to the present invention, step (b) of the method for identifying a compound capable of modulating the activity of the JAK/STAT pathway comprises determining the degree of modulation of the at least one target molecule by the compound. Preferably, the degree of modulation of the at least one target molecule by the compound may be determined either by measuring the amount and/or expression rate of the nucleic acid molecules of (i) or by measuring the amount and/or activity of the polypeptide molecules of (ii). A variety of protocols including, for example, ELISA, RIA, and FACS, for measuring nucleic acid molecules and/or proteins are known in the art and provide a basis for measuring the amount and/or expression rate of a nucleic acid molecule or the amount and/or activity of a polypeptide molecule. Particularly, the capability of a substance to modulate the activity of the JAK/STAT pathway is determined as described in the Example.

According to the present invention, the method for identifying a compound capable of modulating the activity of the JAK/STAT pathway may be a molecular based assay or a cellular assay. Therefore, the at least one target molecule may be provided either *in vivo* in a cellular system, preferably a cellular system overexpressing the at least one target molecule, or *in vitro* in cell fractions containing the at least one target molecule or with the at least one target molecule in a substantially isolated and purified form. Methods for providing the at least one target molecule are well known in the art and may be used in performing the present invention. According to the present invention, it is preferred that the method for identifying a compound capable of modulating the activity of the JAK/STAT pathway is performed in a high-throughput format.

A second aspect of the present invention pertains to the use of at least one molecule selected from
(i) nucleic acid molecules, comprising
   (i.1) a nucleotide sequence as shown in SEQ ID NOs. 88 to 265;
   (i.2) a nucleotide sequence which is complementary to a nucleotide sequence of (i.1);
   (i.3) a nucleotide sequence which has an identity of at least 65 % to a nucleotide sequence of (i.1) or (i.2); and/or
   (i.4) a nucleotide sequence which hybridizes under stringent conditions to a nucleotide sequence of (i.1), (i.2) or (i.3);
   and
(ii) polypeptide molecules
   (ii.1) encoded by the nucleic acid molecules of (i) and/or
   (ii.2) having the sequences as shown in SEQ ID NOs. 1-87,
   as a target for the modulation of the activity of the JAK/STAT pathway.

Within the context of the present invention, the nucleic acid molecules of (i), comprising (i.1) a nucleotide sequence as shown in SEQ ID NOs. 88 to 265, (i.2) a nucleotide sequence which is complementary to a nucleotide sequence of (i.1), (i.3) a nucleotide sequence which has an identity of at least 65 % to a nucleotide sequence of (i.1) or (i.2), and/or (i.4) a nucleotide sequence which hybridizes under stringent conditions to a nucleotide sequence of (i.1), (i.2) or (i.3), and the polypeptide molecules of (ii) encoded by the nucleic acid molecules of (i) used as afore-mentioned are as described above.

A third aspect of the present invention relates to a method for modulating the activity of the JAK/STAT pathway comprising contacting a cell with at least one molecule selected from
(i) nucleic acid molecules, comprising
   (i.1) a nucleotide sequence as shown in SEQ ID NOs. 88 to 265;
   (i.2) a nucleotide sequence which is complementary to a nucleotide sequence of (i.1);
   (i.3) a nucleotide sequence which has an identity of at least 65 % to a nucleotide sequence of (i.1) or (i.2); and/or
   (i.4) a nucleotide sequence which hybridizes under stringent conditions to a nucleotide sequence of (i.1), (i.2) or (i.3);
(ii) polypeptide molecules
   (ii.1) encoded by the nucleic acid molecules of (i) and/or
   (ii.2) having the sequences as shown in SEQ ID NOs. 1-87;
   and
(iii) effector molecules of (i) and/or (ii).

The method for modulating the activity of the JAK/STAT pathway may suitably be performed as molecular based assay or cellular assay. Preferably, the cell used in the method for modulating the activity of the JAK/STAT pathway is a cell showing the JAK/STAT pathway, e.g. an animal cell.

According to the present invention, the nucleic acid molecules of (i), comprising (i.1) a nucleotide sequence as shown in SEQ ID NOs. 88 to 265, (i.2) a nucleotide sequence which is complementary to a nucleotide sequence of (i.1), (i.3) a nucleotide sequence which has an identity of at least 65 % to a nucleotide sequence of (i.1) or (i.2), and/or (i.4) a nucleotide sequence which hybridizes under stringent conditions to a nucleotide sequence of (i.1), (i.2) or (i.3), and the polypeptide molecules of (ii) encoded by the nucleic acid molecules of (i) used according to the method for modulating the activity of the JAK/STAT pathway are as described above.

Moreover, the effector molecules of (i) and/or (ii) used according to the method for modulating the activity of the JAK/STAT pathway are selected from polypeptides such as proteins, enzymes, antibodies, polypeptide inhibitors, polypeptide activators, agonist, antagonists, mimetics, oligopeptides, cofactors, low molecular weight substances, antisense molecules, RNAi molecules and ribozymes. Preferably, the effector molecules of (i) and/or (ii) are compounds identified by the method for identifying compounds of modulating the activity of the JAK/STAT pathway described above. More preferably, the effector molecules of (i) and/or (ii) are antibodies or fragments thereof directed against a polypeptide molecule of (ii), antisense molecules directed against a nucleic acid molecule of (i) and/or RNAi molecules.

Further, the present invention is concerned in a fourth aspect with a pharmaceutical composition comprising as an active agent at least one molecule selected from
(i) nucleic acid molecules, comprising
   (i.1) a nucleotide sequence as shown in SEQ ID NOs. 88 to 265;
   (i.2) a nucleotide sequence which is complementary to a nucleotide sequence of (i.1);
   (i.3) a nucleotide sequence which has an identity of at least 65 % to a nucleotide sequence of (i.1) or (i.2); and/or
   (i.4) a nucleotide sequence which hybridizes under stringent conditions to a nucleotide sequence of (i.1), (i.2) or (i.3);
(ii) polypeptide molecules
   (ii.1) encoded by the nucleic acid molecules of (i) and/or
   (ii.2) having the sequences as shown in SEQ ID NOs. 1-87;
   and
(iii) effector molecules of (i) and/or (ii).

According to the present invention, the nucleic acid molecules of (i), comprising (i.1) a nucleotide sequence as shown in SEQ ID NOs. 88 to 265, (i.2) a nucleotide sequence which is complementary to a nucleotide sequence of (i.1), (i.3) a nucleotide sequence which has an identity of at least 65 % to a nucleotide sequence of (i.1) or (i.2), and/or (i.4) a nucleotide sequence which hybridizes under stringent conditions to a nucleotide sequence of (i.1), (i.2) or (i.3), the polypeptide molecules of (ii) encoded by the nucleic acid molecules of (i) and the effector molecules (of (iii)) of (i) and/or (ii) comprised in the pharmaceutical composition of the invention are as described above.

In addition to the at least one active ingredient, the pharmaceutical composition of the invention may contain suitable pharmaceutically acceptable carriers, diluents and/or adjuvants, which facilitate processing of the active ingredient into preparations, which can be used pharmaceutically. Further details on techniques for formulation and administration may be found in the latest edition of Remington's Pharmaceutical Sciences (Maack Publishing Co., Easton, Pa.).

The pharmaceutical composition of the present invention is particularly suitable for the diagnosis, prevention or treatment of a JAK/STAT pathway associated disorder. Preferably, the JAK/STAT pathway associated disorder is selected from the group consisting of papillary thyroid carcinoma, Refsum disease, blood-brain barrier glucose transport defect, X-linked nonsyndromic mental retardation, long QT syndrome 4, subcortical laminar heterotopia, leukemia, steroid-resistant nephrotic syndrome, invasive pituitary tumor, sporadic Sotos syndrome, autosomal dominant iron overload, hereditary pancreatitis, stomatocytosis I, atypical Rett syndrome, phosphoglycerate dehydrogenase deficiency, Wolman disease, neurophysiologic defect in schizophrenia, autosomal recessive SCID (T-negative/B-positive type), atelostogenesis (type I), Larson syndrome, spondylocarpotarsal synostosis syndrome, frontometaphyseal dysplasia, diabetes mellitus (type II), susceptibility to insulin resistance, Griscelli Syndrome, limb-girdle muscular dystrophy (type 2A), growth hormone insensitivity with immunodeficiency and breast cancer.

In one embodiment of the present invention the pharmaceutical composibon is used for the prevention or treatment of a JAK/STAT pathway associated disorder. Pharmaceutical compositions suitable for the prevention or treatment of a JAK/STAT pathway associated disorder include compositions wherein the at least one active ingredient is contained in an effective amount to achieve the intended purpose. The determination of an effective dose is well within the capability of those skilled in the art. For any compounds, the therapeutically effective dose can be estimated initially either in cell culture assays or in animal models, usually mice, rabbits, dogs or pigs. The animal model may also be used to determine the appropriate concentration range and route of administration. Such information can then be used to determine useful doses and routes for administration in humans.

The actual amount of the pharmaceutical composition administered, will of course, be dependent on the subject being treated, on the subject's weight, the severity of the JAK/STAT pathway associated disorder, the manner of administration and the judgement of the prescribing physician. For the pharmaceutical composition of the invention, a daily dosage of 1 to 200 mg of the at least one active ingredient per kg and day, particularly 10 to 100 mg of the at least one active ingredient per kg and day, is suitable. Suitable routes of administration may, for example, include oral, intravenous, intramuscular, intraarterial, intramedullary, intrathecal, intraventricular, transdermal, subcutaneous, intraperitoneal, intranasal, enteral, topical, sublingual or rectal administrations. Preferably, the subject being treated is an animal, in particular a human being.

In another embodiment of the present invention the pharmaceutical composition is used for the diagnosis of a JAK/STAT pathway associated disorder, e.g. a disorder characterized by or associated with the over- or underexpression of a nucleic acid molecule of (i) or a polypeptide molecule of (ii). Diagnostic assays include methods which utilize the pharmaceutical composition and a label to detect the nucleic acid molecule of (i) or polypeptide molecule of (ii) in human body fluids or extracts of cells or tissues.

Finally, a further aspect of the present invention relates to the use of at least one molecule selected from
(i) nucleic acid molecules, comprising
   (i.1) a nucleotide sequence as shown in SEQ ID NOs. 88 to 265;
   (i.2) a nucleotide sequence which is complementary to a nucleotide sequence of (i.1);
   (i.3) a nucleotide sequence which has an identity of at least 65 % to a nucleotide sequence of (i.1) or (i.2); and/or
   (i.4) a nucleotide sequence which hybridizes under stringent conditions to a nucleotide sequence of (i.1), (i.2) or (i.3);
(ii) polypeptide molecules
   (ii.1) encoded by the nucleic acid molecules of (i) and/or
   (ii.2) having the sequences as shown in SEQ ID NOs. 1-87;
   and
(iii) effector molecules of (i) and/or (ii);
for the manufacture of a pharmaceutical composition for the diagnosis, prevention or treatment of a JAK/STAT pathway associated disorder.

According to the present invention, the nucleic acid molecules of (i), comprising (i.1) a nucleotide sequence as shown in SEQ ID NOs. 88 to 265, (i.2) a nucleotide sequence which is complementary to a nucleotide sequence of (i.1), (i.3) a nucleotide sequence which has an identity of at least 65 % to a nucleotide sequence of (i.1) or (i.2), and/or (i.4) a nucleotide sequence which hybridizes under stringent conditions to a nucleotide sequence of (i.1), (i.2) or (i.3), the polypeptide molecules of (ii) encoded by the nucleic acid molecules of (i) and the effector molecules (of (iii)) of (i) and/or (ii) used according to the present invention for the manufacture of a pharmaceutical composition for the diagnosis, prevention or treatment of a JAK/STAT pathway associated disorder are as described above.

Moreover, according to the present invention, the pharmaceutical composition and the JAK/STAT pathway associated disorder are as described above.

Methods for the manufacture of a pharmaceutical composition, comprising the step of admixing at least one molecule selected from nucleic acid molecules of (i), comprising (i.1) a nucleotide sequence as shown in SEQ ID NOs. 88 to 265, (i.2) a nucleotide sequence which is complementary to a nucleotide sequence of (i.1), (i.3) a nucleotide sequence which has an identity of at least 65 % to a nucleotide sequence of (i.1) or (i.2), and/or (i.4) a nucleotide sequence which hybridizes under stringent conditions to a nucleotide sequence of (i.1), (i.2) or (i.3), polypeptide molecules of (ii) encoded by the nucleic acid molecules of (i) and effector molecules (of (iii)) of (i) and/or (ii) with a pharmaceutically acceptable excipient, vehicle or carrier and optionally other ingredients are well known to those skilled in the art and may be used in performing the present invention.

Further, the present invention shall be explained by the following Tables, Figures and Example.

### Tables

**Table 1** shows the RNAi JAK/STAT phenotypes.
**Table 2** shows the functional groups classified by InterPro prediction and GO.
**Table 3** shows the genetic interactions with *hop^{Tuml}.*
**Table 4** shows sequence and cytological information.
**Table 5** shows human homologues of *Drosophila* genes with JAK/STAT phenotypes.
**Table 6** shows human disease homologues of *Drosophila* genes with JAK/STAT phenotypes.
**Table 7** shows the expected and observed phenotype frequency.

### Figures

**Fig. 1** shows the genome-wide RNAi screen for JAK/STAT signalling factors. **a:** Schematic representation of the *Drosophila* JAK/STAT signalling pathway. **b:** Knock-down of known JAK/STAT components leads to loss of pathway induction by Upd whereas knock-down of *lacZ, toll* and *relish* show no effect. The red line indicates a 70-fold reporter induction relative to negative control dsRNA. Error bars represent standard deviations of six experiments. **c**: Screening approach. 20,026 dsRNA were screened in duplicate in 384-well plates prior to computational analysis and retesting. FL: firefly luciferase (indicated in red); RL: *Renilla* luciferase (indicated in yellow) **d**: Q-Q plot of normally distributed quantiles against actual screening result quantiles in the pathway reporter channel. A perfect fit to a normal distribution is represented by the red line. Tails of positively and negatively interacting dsRNAs at each extreme with a *z*-score threshold of > 2 and < -2 represent RNAi experiments with significant phenotypes (p<0.05).

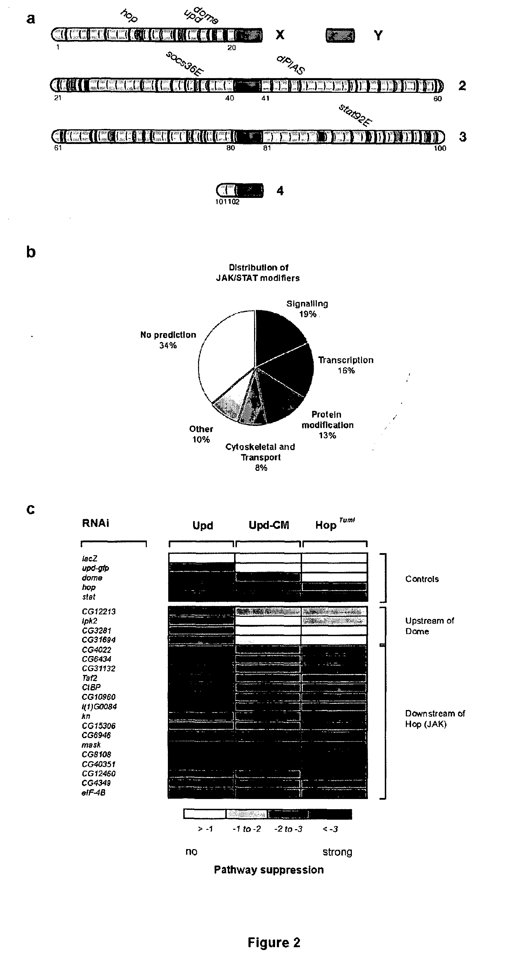

**Fig. 2** shows the analysis of JAK/STAT activity modulators. **a**: Schematic representation of positive (red) and negative (green) regulator loci distributed within the *Drosophila* genome. An interactive version of this panel is available at http://www.dkfz.de/signaling/jak-pathway/cytomap.php. **b**: Distribution of predicted gene functions. **c**: Epistasis analysis of the indicated positive pathway regulators showing interactions graded from none (yellow) to strong (red). Results shown have been obtained in two independent octuplicate experiments. Upd: Upd ectopic expression; Upd-CM: Upd conditioned medium; hop^{Tuml}: expression of a constitutively active JAK-allele. Colour coding of z-scores is shown in the key.

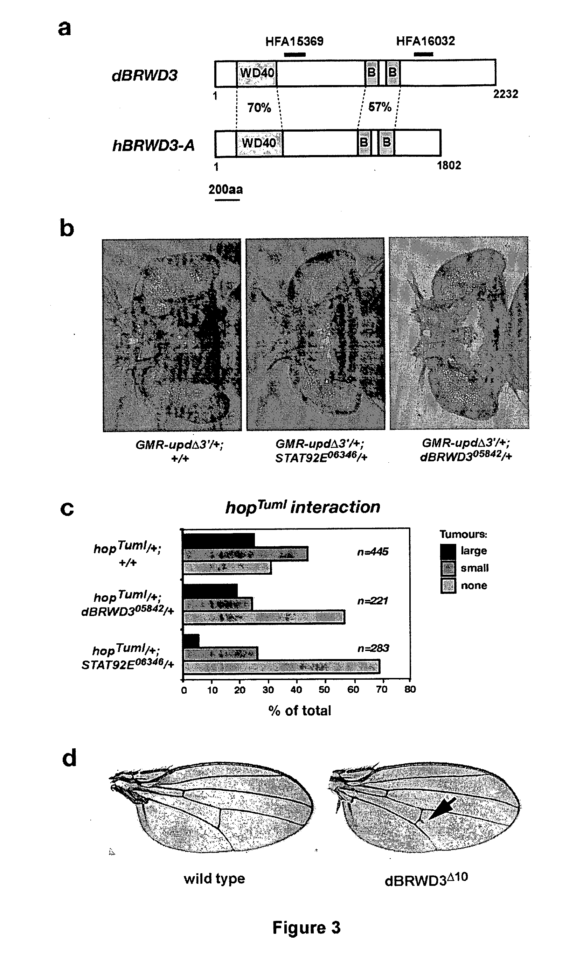

**Fig. 3** shows that dBRWD3 functions as a JAK/STAT pathway component. **a**: Domain structure and sequence similarity of *Drosophila* and human BRWD3 proteins. Percentages show the similarity in the amino acid sequence and regions targeted by two independent dsRNAs independently recovered in the screen are shown. **b**: Adult *Drosophila* heads heterozygous for the *GMR-updΔ3'* transgene crossed to wild type (left), *stat92E* (middle) and *dBRWD3* mutants (right). Note the strong reduction in eye size following removal of pathway components. **c**: *hop^{Tuml}* induced tumour formation is significantly decreased in both size and frequency of tumours in *stat92E* and *dBRWD3* heterozygous backgrounds. **d:** By comparison to adult wild type wings (left), ectopic wing vein material (arrow) is present in homozygous *dBRWD3^{Δ10}* mutant (a putative hypomorphic allele, right), a phenotype reminiscent of the *stat92E^{HJ}* mutant.

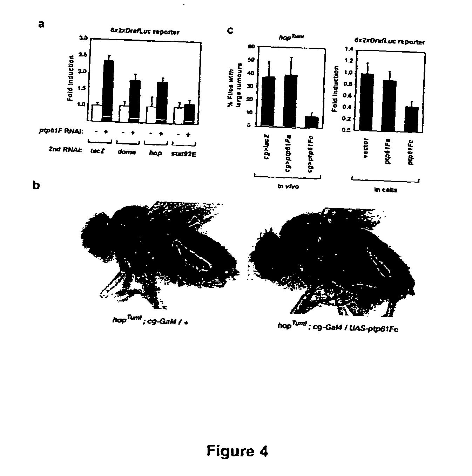

**Fig. 4** shows thatPtp61F is a tumour suppressor *in vivo.* **a:** Epistasis analysis of *ptp61F* dsRNA in cell culture revealed that it acts downstream of Hop and upstream or parallel to STAT92E. **b:** Haemocyte specific misexpression of *ptp61F* can protect *hop^{Tuml}* mutants from melanotic tumour formation. Compare large black tumours in controls (arrow heads, left) with small tumours present in *ptp61F* expressing individual (right). **c:** Quantitative analysis of large tumour formation in *hop^{Tuml}* mutants expressing cytoplasmic Ptp61Fa and nuclear Ptp61Fc shows specificity of rescue for the nuclear isoform (left), an effect that is mirrored by over-expression of the same isoforms in tissue culture based reporter assays (right). Error bars represent standard deviations of 3 or 4 independently tested transgenic lines or eight parallel cell culture experiments.

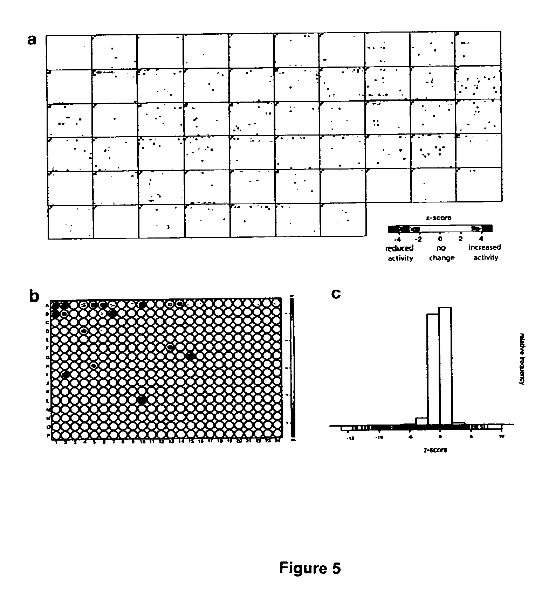

**Fig. 5** shows an overview of primary RNAi screen data. **a:** False colour representation of the genome-wide screen showing averaged z-scores for each well present in the fifty seven 384-well duplicate plates. Key indicates the colours associated with the z-scores: -4 (red) represents a strong decrease in reporter activity, +4 (blue) represents an increase in activity. Four controls were included in the top left corner of each plate and are visible in all plates except 1 and 9 for which these dsRNA controls failed. **b:** False colour representation of average z-scores for a representative example from the genome-wide screen (plate 34). Controls present in the lop left corner of each plate were *hop* (A1), *dome* (A2), *stat92E* (B1) and *socs36E* (B2). dsRNAs from the library were present in all other wells including position B07 which targets *hopscotch.* L10 which targets CG2033 was excluded from the final list because of a cell viability phenotype previously identified in both Kc and S2R+ cells (Boutros, M. et al., Science 303, 832-5 (2004)). Similarly, 102 and G20 (which both target *sbr*/*CG17335*) were excluded due to variability in retesting and a previously described bi-nucleate phenotype (Kiger, A. A. et al., J Biol 2, 27 (2003)). Colour coding for z-scores is shown in the key and uses the same scheme shown in a. c: Histogram of z-scores for the genome-wide screen indicates that the majority of dsRNA experiments do not modify JAK/STAT signaling activity.

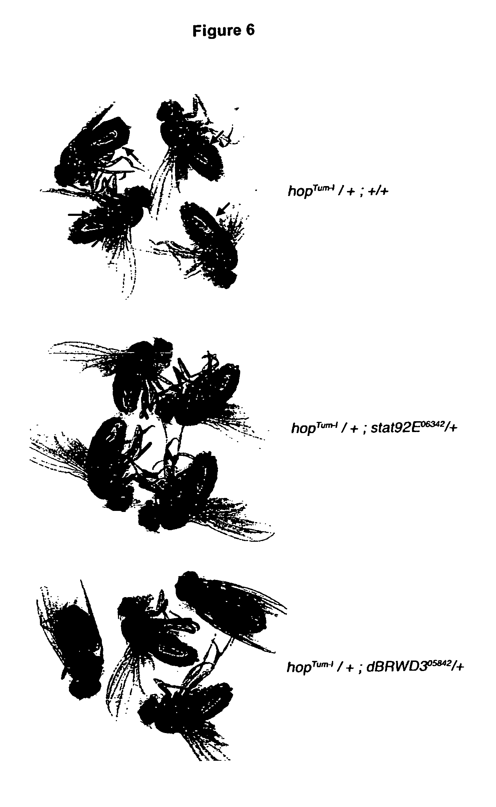

**Fig. 6** shows the loss of JAK/STAT pathway components and *hop^{Tuml}* induced tumour formation. *hop^{Tuml}*/*+;* +/+ females (top) frequently contain large black melanotic tumours (arrows). *hop^{Tuml}*/*+;stat92E⁰⁶³⁴²*/*+* heterozygotes which lack one copy of *stat92E* (middle) contain fewer and smaller tumours. *hop^{Tuml}*/*+;dBRWD3⁰⁵⁸⁴²*/*+* (bottom) also contain fewer and smaller tumours. Flies were grown in parallel independent experiments at 25 °C and are representative examples of the individuals recovered (see Table 3 for further information).

### Example

Signalling pathways mediating the transduction of information between cells are essential for development, cellular differentiation and homeostasis (Brivanlou, A. H. & Darnell, J. E., Jr., Science 295, 813-8. (2002)). Their dysregulation is also frequently associated with human malignancies. The JAK/STAT pathway represents one such signalling cascade whose evolutionarily conserved roles include cell proliferation and haematopoiesis (Hombria, J. C. & Brown, S., Curr Biol 12, R569-75 (2002)). Here, the inventors of the present invention describe a systematic genome-wide survey for genes required for JAK/STAT pathway activity. Analysis of 20,026 RNAi-induced phenotypes in cultured *Drosophila melanogaster* haemocyte-like cells identified interacting genes encoding 4 known and 84 previously uncharacterised proteins. Subsequently, cell based epistasis experiments have been used to classify these based on their interaction with known components of the signalling cascade. In addition to multiple human disease gene homologues, the inventors of the present invention have identified the tyrosine phosphatase Ptp61F and the *Drosophila* homologue of *BRWD3,* a bromo-domain containing protein disrupted in leukaemia (Kalla, C. et al., Genes Chromosomes Cancer 42, 128-43 (2005)). Moreover, *in vivo* analysis demonstrates that disrupted *dBRWD3* and overexpressed *Ptp61F* function as suppressors of leukaemia-like blood cell tumours. This screen represents a comprehensive identification of novel loci required for JAK/STAT signalling and provides molecular insights into an important pathway relevant for human diseases.

### 1. Experimental procedures

### 1.1. Constructs and pathway reporter

The JAK/STAT firefly luciferase reporter *6x2xDrafLuc* was constructed by multimerisation of a molecularly characterised STAT92E binding site present in the promoter of the endogenous target genes Draf (Kwon, E. J. et al., J Biol Chem 275, 19824-19830 (2000)) while the *4xsocsLuc* reporter is based on a single region containing four potential STAT92E binding sites present within the first intron of *socs36E* (Karsten, P., Hader, S. & Zeidler, M. P., Mech Dev 117, 343-6 (2002)). A *Renilla* luciferase reporter gene under the control of the constitutively active *Actin5C* promoter was co-transfected and used to monitor cell number.

Strictly speaking, the JAK/STAT reporter *6x2xDrafLuc* was constructed by multimerisation of STAT92E binding sites. Specifically, a 165 bp blunted BamHl/Xbal fragment from the original *p2xDrafSTAT(wt)* (Kwon, E. J. et al., J Biol Chem 275, 19824-19830 (2000)) (a kind gift of M. Yamaguchi and M.A. Yoo) was inserted into the Smal cut *p2xDrafSTAT(wt).* The same fragment was amplified by PCR with Notl sites on both ends and inserted into compatible sites to yield the *3x2xDrafLuc* reporter containing six STAT92E binding sites. These fragments were amplified again and the resulting 540 bp fragment was inserted into the Sacll cut *3x2xDrafLuc* vector to generate the *6x2xDrafLuc* reporter with an enhancer of approximately 1000 bp containing a total of 12 STAT92E binding sites. A second independent JAK/STAT pathway reporter, *4xsocsLuc,* was generated by amplifying a 745 bp product from genomic DNA using the primers 5'-GTTAGGTACCGGGTCGCAGTATCGTTGGCG-3' and 5'-CGAAGGATCC CTGTCACTTCTCAGAAATCGGTC-3'. This was then cut with EcoRI/BamHI to give a 285 bp fragment, subcloned into pBS(KS+) (Stratagene) and re-excised with Asp718/BamHl. This 340 bp fragment, containing four predicted STAT92E binding sites (Karsten, P., Hader, S. & Zeidler, M., Mech Dev 117, 343. (2002)), was cloned into Asp718/BgIII sites of pGL3 vector (Promega).

The *pAct-RL* vector expressing *Renilla* luciferase from a constitutive reporter was generated by cloning a 974 bp fragment coding for *Renilla* luciferase from pRLSV40 (Invitrogen) into the BamHI/Xbal cut pP_{Ac5c}-PL vector (a kind gift from Dan Curtis). To generate the *pAct-UpdGFP* vector, a cDNA coding for Upd (Harrison, D. A., Binari, R., Nahreini, T. S., Gilman, M. & Perrimon, N., Embo J 14, 2857-65 (1995)) was fused in frame to EGFP via a BamHI site and inserted into the BamHI/XbaI cut pP_{Ac5c}-PL vector. A vector expressing the dominant gain-of-function allele Hop^{TumL} was cloned by inserting the open reading frame obtained from *pUAS-hop^{TumL}* (Harrison, D. A., Binari, R., Nahreini, T. S., Gilman, M. & Perrimon, N., Embo J 14, 2857-65 (1995)) into the Notl/Xbal cut pAc5.1A vector (Invitrogen). A *pAc5.1-Sid-1* expression construct which was used to facilitate uptake of dsRNA was a gift of Craig Hunter (Feinberg, E. H. & Hunter, C. P., Science 301, 1545-7 (2003)).

To generate Ptp61F expression constructs, cDNAs encoding Ptp61Fc (LP01280) and Ptp61Fa (RE01370) were obtained from the *Drosophila* Genomics Resource Center (University of Indiana). cDNA clones were analysed by restriction analysis and end sequencing to confirm their integrity before subcloning into pAc5.1A and pUAST (Brand, A. H. & Perrimon, N., Development 118, 401-15 (1993)). For Ptp61Fc, the coding region of LP01280 was excised as an EcoRI/XhoI (partial digest) fragment of 1.8kb and cloned into pUAST. Subsequently, the insert was re-excised with EcoRI/Xbal and cloned into pAc5.1A (Invitrogen). For Ptp61Fa, the coding region of RE01370 was cut out with EcoRI/Asp718(filled) and cloned into pAc5.1A cut EcoRI/Xbal(filled). The generate a pUAST construct, an EcoRI/Asp718 fragment was used.

To clone *p[w⁺,UAS-dPIAS-GFP],* the EST clone LD09022 was used as a template in conjunction with the oligos 5'-CATCGGATCCTGCAAAAAGGGG TCCAACGTACC GGAT-3' and 5'-GGGGTACCAAAAATGGTGCATATGCTT CGA-3' to amplify a region coding for 522 amino acids. The resulting product was sequenced, cut with Asp718/BamHl and subcloned into pBS-EGFP-B to generate an in frame C-terminal EGFP fusion protein. This gene was then subcloned as an Asp718/Xbal fragment into pUAST (Brand, A. H. & Perrimon, N., Development 118, 401-15 (1993)).

Multiple independent transgenic *Drosophila* stocks of each transformation vector construct were generated by microinjection of embryos using standard techniques (Spradling, A. C. & Rubin, G. M., Science 218, 341-347 (1982)).

### 1.2. Genome-wide RNAi screening

A genome-wide RNAi library based on PCR templates with an average length of 408bp flanked by T7-promotor binding sites was generated by *in vitro* transcription (Boutros, M. et al., Science 303, 832-5 (2004)). Therefore, PCR fragments containing T7 promoter sequences on each end (Hild, M. et al., Genome Biol 5, R3 (2003)) were used as templates to generate 20,026 dsRNAs by *in vitro* transcription (Boutros, M. et al., Science 303, 832-5 (2004)). After DNAse I treatment, dsRNAs were purified by ethanol precipitation and individually quality controlled by gel electrophoresis. RNAs were diluted to a working stock concentration and aliquoted in ready-to-screen 384-well tissue culture plates (Greiner). Computational mapping predict that the 20,026 RNA fragments target > 91 % of all predicted genes in the *Drosophila* genome (Annotation 4.0) (Misra, S. et al., Genome Biol 3, RESEARCH0083-3 (2002)). Protocols and supplemental material can be found at http://www.dkfz-heidelberg.de/signaling/jak-pathway/. Complete primer and amplicon sequence information for double-stranded RNAs including calculation of predicted efficiency and off-target effects for the RNAi library is publicly accessible at http://rnai.dkfz.de.

For screening experiments, *Drosophila* Kc₁₆₇ cells were maintained in Schneider's medium (invitrogen) supplemented with 10 % foetal bovine serum (PAA) and 100 µg/ml penicillin-streptomycin (Invitrogen). Cells were grown at 25°C at subconfluent densities. The RNAi screening experiments were performed in white, polystyrene 384-well tissue culture plates (Greiner 781 073). A total of fiftyseven 384-well screening plates were loaded with an average of 75 nM (500 ng) dsRNA in 5 µl of 1 mM Tris pH 7. Kc₁₆₇ cells were transfected in batch in 6-well plates with 0.25 µg of the *6x2xDrafLuc* JAK/STAT signalling reporter, 0.6 µg of *pAct-UpdGFP* expression vector, 0.25 µg *pAc5.1-Sid-1* (to facilitate RNA uptake (Feinberg, E. H. & Hunter, C. P., Science 301, 1545-7 (2003))) and 0.025 µg of *pAct-RL* vector as a co-reporter. The total plasmid amount was normalised to 2 µg with a *pAc5.1* plasmid (Invitrogen) and 5x10⁶ cells were transfected with Effectene (Qiagen). After 7 hours incubation at 25°C, batch transfected cells were resuspended in serum-free medium. Subsequently 15,000 cells in 20 µl were dispensed per dsRNA containing well using an automated liquid dispenser (MultiDrop, Thermo Labsystems). Cells were incubated for 45 min and 30 µl of serum-containing medium was added to each well. Cells were grown for 5 days to allow for protein depletion. Pathway activity was measured for using a luminescence assay for firefly and *Renilla* luciferase on a Mithras LB940 plate reader (Berthold Technologies). Luminescence of the *Renilla* luciferase was measured using a 490 nm filter set. Screens were performed in duplicate. Each plate contained dsRNA targeting *stat92E, dome, hop* and *socs36E* in A1, A2, B1, B2 which were used as positive controls (see also Fig. 5b). For retests, an independent JAK/STAT pathway reporter (*4xsocsLuc*) was used which contained a STAT-binding site from the endogenous JAK/STAT-pathway target *socs36E* (Karsten, P., Hader, S. & Zeidler, M., Mech Dev 117, 343. (2002)).

To identify candidate genes that significantly increase or decrease JAK/STAT signalling pathway activity, the raw luciferase results were normalised by median centering of each 384-well plate (separately by channel). Z-scores were calculated as the number standard deviation that a particular well differed from the median of the 384-well plate. To minimise false negatives, the inventors of the present invention applied a set of low-stringency criteria to generate a list of candidate genes to be used in specific retests. First, the inventors filtered dsRNA treatments with z-scores > 2 for negative regulators or < -2 for positive regulators, respectively. Treatments that showed a high variability between duplicates were excluded. Further, RNAi experiments that showed z-scores of > 2 or < -2 in the control channel were not selected for retesting. The inventors also filtered against previously identified cell viability modifiers that show a phenotype in cultured *Drosophila* cells (Boutros, M. et al., Science 303, 832-5 (2004)). The inventors also excluded genes that showed phenotypes in other screens. These filtering steps led to a final list of approximately 107 candidates that were selected for retesting. New dsRNA was re-synthesized as described above and repeat assays were performed in quadruplicate. 89 of the candidates were confirmed using a second JAK/STAT reporter assay (*4xsocsLuc*) employed to exclude reporter-specific artefacts. Data analysis and representation were performed using R and Bioconductor (Gentleman, R. C. et al., Genome Biol 5, R80 (2004)).

The predicted genes targeted by 91 dsRNAs were classified according to InterPro (Mulder, N. J. et al., Nucleic Acids Res 33 Database Issue, D201-5 (2005)) and GO (Harris, M. A. et al., Nucleic Acids Res 32, D258-61 (2004); Drysdale, R. A. et al., Nucleic Acids Res 33 Database Issue, D390-5 (2005)) and manual inspection was used to order genes into functional groups. Predicted proteins without InterPro domain or GO annotation were classified as ''Unknown" although these sequences might encode structurally conserved proteins. To determine whether *Drosophila* proteins have homologues in other species, the inventors used BLASTP searches against the protein predictions from *H. sapiens* (NCBI build 35) with a cut-off of E < 10⁻¹⁰. Databases were obtained from Ensembl (http://www.ensembl.org) (Clamp, M. et al., Nucleic Acids Res 31, 38-42 (2003)) and Flybase (http://www.flybase.org) (Drysdale, R. A. et al., Nucleic Acids Res 33 Database Issue, D390-5 (2005)). Reciprocal best BLASTP analysis was used to identify the human homologue of CG31132. CG31132 and human BRWD3 are classified as orthologous pairs by InParanoid (http://inparanoid.cgb.ki.se/).

### 1.3. Cell-Based Epistasis Experiments

To undertake epistasis experiments, cells were transfected with vectors to stimulate pathway activity (see below) for 7 hours and 30,000 cells in 50 µl of serum-free medium were seeded into wells of clear bottom 96-well plates (Greiner), which contained 1.5 µg of the dsRNAs to be tested (listed in Fig. 2c). Following 1 hour incubation, 75 µl medium supplemented with 10 % foetal bovine serum was added to the cells, plates were sealed and cells lysed after 5 days to measure luciferase activities.

Each dsRNA was tested for its ability to suppress pathway activity under three conditions: (1) in Upd-expressing cells (screening conditions). (2) in cells treated with Upd-conditioned medium (Upd-CM), and (3) in cells expressing the activated form of JAK, Hop^{Tuml} (Harrison, D. A., Binari, R., Nahreini, T. S., Gilman, M. & Perrimon, N., Embo J 14, 2857-65 (1995); Luo, H., Hanratty, W. P. & Dearolf, C. R., Embo J 14, 1412-20 (1995)). Specifically, for Upd overexpression 5x10⁶ Kc₁₆₇ cells were transfected with 600 ng *pAct-UpdGFP,* 500 ng *6x2xDrafLuc* reporter, 250 ng *pAc5.1-Sid-1,* 25 ng *pAct-RL* and *pAc5.1* to a total of 2 µg DNA. For Hop^{TumL} overexpression, 5x10⁶ Kc₁₆₇ cells were transfected with 200 ng *pAct-hop^{TumL},* 500 ng *6x2xDrafLuc* reporter, 250 ng *pAc5.1-Sid-1,* 25 ng *pAct-RL* and *pAc5.1* to a total of 2 µg DNA. To analyse processes upstream of Upd, two batches of cells were transfected separately to generate 'responder' and 'Upd-producer' cells. The 'responder' cells were batch transfected with 500 ng *6x2xDrafLuc* reporter, 250 ng *pAc5.1-Sid-1,* 25 ng *pAct-RL* and *pAc5.1* to a total of 2 µg plasmid DNA and subsequently seeded into 96-well plates containing the respective dsRNAs as described above. The 'Upd-producing' cells were transfected with 2 µg *pAct-UpdGFP* and cultured in 10 cm dishes (Falcon). Three days after transfection, cells were treated with 50 µg/ml Heparin (Sigma). After 24 hours, the supematant was harvested, cleared by centrifugation and passed through a 0.2 µm filter (Millipore). 50 µl of this Upd-conditioned medium were then used to stimulate pathway activity in the 'responder' cells for 24 hours. Control medium from untransfected Heparin treated cells did not elicit pathway activity (not shown).

Experiments were performed in eight replicates and repeated at least twice. Reporter activity in the firefly luciferase channel was divided by the *Renilla* luciferase channel to normalise for cell number. Z-scores were calculated as the multiples of the standard deviation that a specific RNAi treatment differed from cells treated with *lacZ* dsRNA as negative controls. Z-scores were subsequently transformed into a false-colour representations as depicted in Fig. 2c.

RNA controls as shown in Fig. 2c were *in vitro* transcribed from PCR templates generated using the following gene-specific primer sequences: *5T7lacZ*: GAATTAATACGACTCACTATAGGGAGACAGTGGCGTCTGGCGGAAAA (SEQ ID NO. 448), *3T7lacZ:* GAATTAATACGACTCACTATAGGGAGATCCGAGC CAGTTTACCCGCT (SEQ ID NO. 449), 5T7*gfp*: TAATACGACTCACTATAGGACG GCCGCCATTAACAAGCAAAAG (SEQ ID NO. 450) and 3T7*gfp*: TAATACGACTCA CTATAGGCTGGGCGGAGCGGATGATG (SEQ ID NO. 451). Note that the *gfp* dsRNA was used to target the Upd-GFP transgene and leads to a loss-of pathway activity. *lacZ* dsRNA was used as a negative control.

For epistasis analysis of the putative negative regulator *ptp61F,* cells were batch transfected with reporter and Upd inducer as described above. Subsequently, these cells were treated with 1.5 µg of dsRNA targeting the *ptp61F* transcript and 1.5 µg of dsRNA against *lacZ, dome, hop* or *stat92E.* In parallel, cells from the same transfection batch were treated with *lacZ, dome, hop* or *stat92E* dsRNAs alone. After normalisation, the values of experiments with control dsRNA alone were set to one. To examine the JAK/STAT phenotype of *ptp61F* in cells, 5x10⁶ Kc₁₆₇ cells were transfected with 0.6 µg *pAct-UpdGFP,* 0.5 µg *6x2xDrafLuc* reporter, 0.25 µg *pAc5.1-Sid-1,* 0.025 µg *pAct-RL* and *pAc5.1* to a total of 2 µg DNA. To assess the effects of the different Ptp61F splice forms, cells were transfected as described before with additional 0.5 µg of *pAct-Ptp61Fa, pAct-Ptp61Fc* or vector control, respectively. JAK/STAT pathway activation was expressed in relation to control cells.

### 1.4. Genetics

A P-element insertion termed *I(3)05842* (Spradling, A. C. et al., Genetics 153, 135-77 (1999)) was identified in the fourth intron of *dBRWD3*/*CG31132* as part of a Flybase search (Drysdale, R. A. et al., Nucleic Acids Res 33 Database Issue, D390-5 (2005)). A *I(3)05842* stock was obtained from the Bloomington stock centre (University of Indiana). The P-element insertion *I (3)05842* is homozygous lethal and fails to complement the *Df(3R)crb87-4* and *Df(3R)crb87-5* deficiencies. Twenty three independent stocks in which the *ry*⁺ marker present in the *P[ry⁺,PZ]* insertion had been lost following a cross to a transposase source were established. Of these, seven were viable revertants (30 %) and include two stocks with the wing vein phenotype (Fig 3d), two are semi-lethal with occasional escapers and the remainder were lethal.

For genetic interaction assays, females of the stock *y,w,hop^{Tuml}*/ *FM7; P [w⁺,cg-Gal4.A]2* (Harrison, D. A., Binari, R., Nahreini, T. S., Gilman, M. & Perrimon, N., Embo J 14, 2857-65 (1995)) were crossed to wild type controls (*OreR* and *w¹¹¹⁸*) and mutations in *stat92E* and *I(3)05842.* The haemocyte specific Gal4 driver line *P[w⁺,cg-Gal4.A]2* allowed specific UAS insertions to be tested for their potential influence on tumour formation. Transgenic animals expressing *UAS-EGFP* or *UAS-β-galactosidase* were used as negative controls while *UAS-dPIAS-EGFP* served as a positive control (Betz, A., Lampen, N., Martinek, S., Young, M. W. & Darnell, J. E., Jr., Proc Natl Acad Sci U S A 98, 9563-8 (2001)) (see Table 3).

Crosses were incubated at 25 °C and adult females heterozygous for the *hop^{Tuml}* chromosome were scored within 24 hours of eclosion for the presence of tumours classified as small (one or two small melanotic spots as shown in Fig. 4b [right]) or large (one or more large melanised growths or more than three small spots; Fig. 4b [left]). Survival rates for *hop^{Tuml}* females appear to be independent of tumour frequency at the time point counted (not shown). Assays were repeated at least twice for each genotype and a representative example from one experiment is shown (Fig. 4b).

Genetic interaction with *P[w⁺,GMR-updΔ3']'19* was undertaken as described in Genetics 165, 1149-66 ((2003), Bach, E. A., Vincent, S., Zeidler, M. P. & Perrimon, N.) using *OreR* and *STAT92E⁰⁶³⁴⁶* as negative and positive controls, respectively. Suppression of *P[w⁺,GMR-updΔ3']'19* induced eye overgrowth by *dBRWD3⁰⁵⁸⁴²* was observed in multiple independent experiments in a majority of individuals of the appropriate genotype. *Drosophila* heads were photographed using a Zeiss STEMI 2000-C binocular microscope and Axiocam camera.

### 2. Results and discussion

Developmental genetic screens in *Drosophila* have identified multiple JAK/STAT pathway components on the basis of their segmentation phenotype (Binari, R. & Perrimon, N., Genes Dev 8, 300-12. (1994); Harrison, D. A., McCoon, P. E., Binari, R., Gilman, M. & Perrimon, N., Genes Dev 12, 3252-63. (1998); Hou, X. S., Melnick, M. B. & Perrimon, N., Cell 84, 411-9 (1996)) and subsequent analysis of the pathway has characterised evolutionarily conserved roles during immune responses, haematopoiesis and cellular proliferation (Lagueux, M., Perrodou, E., Levashina, E. A., Capovilla, M. & Hoffmann, J. A., Proc Natl Acad Sci U S A 97, 11427-32. (2000); Boutros, M., Agaisse, H. & Perrimon, N., Dev Cell 3, 711-22. (2002); Meister, M. & Lagueux, M., Cell Microbiol 5, 573-580 (2003); Mukherjee, T., Castelli-Gair Hombria, J. & Zeidler, M. P., Oncogene in press (2005)). The JAK/STAT signalling cascade in *Drosophila* is comprised of the extracellular ligand Unpaired (Upd) (Harrison, D. A., McCoon, P. E., Binari, R., Gilman, M. & Perrimon, N., Genes Dev 12, 3252-63. (1998)), a trans-membrane receptor with homology to the IL6 receptor family termed Domeless (Dome) (Brown, S., Hu, N. & Castelli-Gair Hombria, J., Curr Biol 11, 1700-5. (2001)), a single Janus tyrosine kinase (JAK) called Hopscotch (Hop) (Binari, R. & Perrimon, N., Genes Dev 8, 300-12. (1994)) and the STAT92E transcription factor (Hou, X. S., Melnick, M. B. & Perrimon, N., Cell 84, 411-9 (1996); Yan, R., Small, S., Desplan, C., Dearolf, C. R. & Darnell, J. E., Jr., Cell 84, 421-30 (1996)) (Fig. 1a). Known regulators of JAK/STAT signalling including a family of *SOCS*-like genes (Callus, B. A. & Mathey-Prevot, B.; Oncogene 21, 4812-4821 (2002); Karsten, P., Hader, S. & Zeidler, M. P., Mech Dev 117, 343-6 (2002)), *dPIAS*/*Su(var)2-10* (Betz, A., Lampen, N., Martinek, S., Young, M. W. & Darnell, J. E., Jr., Proc Natl Acad Sci U S A 98, 9563-8 (2001)) and *STAM* (Mesilaty-Gross, S., Reich, A., Motro, B. & Wides, R., Gene 231, 173-86 (1999)) are functionally conserved and were identified based on their homology to components originally characterised in mammalian cell culture studies (Hombria, J. C. & Brown, S., Curr Biol 12, R569-75 (2002)). Although successful in identifying the pathway members Upd, Dome, Hop and STAT92E, it is probable that forward genetic approaches have missed components possibly due to non-saturating mutagenesis, genetic redundancy or phenotypic pleiotropy (Nagy, A., Perrimon, N., Sandmeyer, S. & Plasterk, R., Nat Genet 33 Suppl, 276-84 (2003)).

In order to identify novel pathway components and circumvent limitations of classical genetic screens, the inventors of the present invention have undertaken a genome-wide RNA interference (RNAi) screen, a powerful technique for the identification of new components of diverse cellular pathways (Kamath, R. S. et al., Nature 421, 231-7 (2003); Kittler, R. et al., Nature 432, 1036-40 (2004); Berns, K. et al., Nature 428, 431-7 (2004); Paddison, P. J. et al., Nature 428, 427-31 (2004); Boutros, M. et al., Science 303, 832-5 (2004)). To this end, the inventors devised a quantitative assay for JAK/STAT signalling activity in cultured *Drosophila* cells by multimerising a STAT92E-binding site from the *Draf* promotor (Kwon, E. J. et al., J Biol Chem 275, 19824-19830 (2000)) to generate the *6x2xDrafLuc* firefly luciferase reporter. Given the role for JAK/STAT signalling in haematopoiesis (Meister, M. & Lagueux, M., Cell Microbiol 5, 573-580 (2003)), the inventors used *Drosophila* hemocyte-like Kc₁₆₇ cells due to their endogenous ability to respond to pathway activation (Fig 1b). On transfection of the *6x2xDrafLuc* reporter and a plasmid to constitutively express the ligand Upd, a robust induction of the reporter gene activity was observed (Fig. 1b). The inventors first examined whether depletion of known pathway components by RNAi (Clemens, J. C. et al., Proc Natl Acad Sci U S A 97, 6499-6503 (2000)) modifies JAK/STAT signalling activity in Kc₁₆₇ cells. The inventors assessed the effect of double-stranded (ds) RNAs targeting the mRNA of the genes *dome, stat92E* and *hop,* as well as dsRNAs directed against the negative regulators *socs36E* and *dPIAS.* As shown in Fig. 1b, knock down of JAK/STAT components results in significant changes in reporter activity while reporter activity in uninduced cells remains at low **levels (Fig. 1b).**

The inventors then set out to systematically identify genes required for JAK/STAT signalling by generating a library of 20,026 dsRNAs targeting 91 % of the predicted transcripts in the *Drosophila* genome. Using this library the inventors performed duplicate genome-wide screens as outlined in Fig. 1c and 5. After computational analysis (Fig. 1d), dsRNAs targeting candidates were resynthesised and assayed with an independent reporter, derived from the promoter of the pathway target gene *socs36E* (Karsten, P., Hader, S. & Zeidler, M. P., Mech Dev 117, 343-6 (2002)) to exclude reporter specific artefacts. These approaches confirmed the identification of 71 dsRNAs which decrease pathway activity (targeting putative positive regulators) and 19 dsRNAs which increase pathway activity (putative negative regulators) (see Table 1). While most modifiers are distributed throughout the genome (Fig. 2a), the X chromosome is devoid of negative regulators, a finding which may be linked to the role of the pathway in *Drosophila* sex determination (Sefton, L., Timmer, J. R., Zhang, Y., Beranger, F. & Cline, T. W., Nature 405, 970-3 (2000)).

Based on InterPro and GO annotations, pathway modifiers were classified according to their predicted functions. Signalling factors, enzymes mediating post-translational protein modifications and transcription factors cumulatively represent 47% of the genes identified (Fig 2b). Furthermore, 74% of the identified loci possess human homologues (E-value <10⁻¹⁰), 33% of which have been implicated in human disease (Tables 5 and 6). Examples of genes identified in the screen include *CG11501* encoding a putatively secreted negative regulator of JAK/STAT signalling previously demonstrated to be a pathway target gene (Boutros, M., Agaisse, H. & Perrimon, N., Dev Cell 3, 711-22. (2002)), *enok*/*CG11290* encoding an acetyl-transferase and the *tumor suppressor protein 101*/*CG9712* gene which encodes a ubiquitin conjugating enzyme. The molecular role of these genes in the regulation of JAK/STAT signalling remains to be determined.

A genetic technique to characterise signalling molecules is the determination of their epistatic relationship with respect to defined pathway components. The inventors therefore performed cell-based epistatic assays to determine the pathway response to Upd expression, Upd conditioned medium or expression of the constitutively active JAK allele *hop^{Tuml}* (Harrison, D. A., McCoon, P. E., Binari, R., Gilman, M. & Perrimon, N., Genes Dev 12, 3252-63. (1998); Sefton, L., Timmer, J. R., Zhang, Y., Beranger, F. & Cline, T. W., Nature 405, 970-3 (2000)) while simultaneously targeting a subset of positive regulators. In this way, dsRNA-inactivated genes required upstream in the pathway can be characterised on the basis of their rescue by pathway activation further downstream (Fig. 2c). For example, while depletion of the interferon-related protein encoded by *CG15401* results in down-regulation of signalling stimulated by Upd expression, stimulation by Upd conditioned medium or *hop^{Tuml}* is unaffected (Fig 2c). This suggests that *CG15401* is required for the production and/or activity of the Upd ligand. Conversely, loss of pathway activity resulting from the knock down of *CG18670* and *CG6400* (now annotated as one gene termed CG31132) cannot be rescued by any form of pathway stimulus implying a function downstream of JAK (Fig. 2c). Although this analysis suggests a role for multiple genes upstream of Dome, this classification is based on the lack of interaction observed under the differing experimental conditions and the molecular basis of these results remains to be confirmed.

In order to confirm the function of candidate genes *in vivo,* the inventors tested examples of both positive and negative regulators of the JAK/STAT signalling pathway. One positive regulator mentioned above is *CG31132* which encodes a 2232 amino acid WD40 and bromo-domain containing protein homologous to human *BRDW3* (Fig. 3a). *BRDW3* is a functionally uncharacterised locus recently identified at the break point of t(X;11) (q13;q23) translocations derived from multiple B-cell chronic lymphocytic leukaemia (B-CLL) patients (Kalla, C. et al., Genes Chromosomes Cancer 42, 128-43 (2005)). In the screen, reduction of pathway activity was observed for two independent dsRNAs present in the library that target different regions of the transcript (Fig. 3a).

A previously uncharacterised mutagenic P-element inserted in the fourth intron of *CG31132* (henceforth termed *dBRDW3⁰⁵⁸⁴²*) has been deposited in public stock collections as part of the *Drosophila* genome project and remobilisation of this transposon indicates that the insertion is responsible for late embryonic lethality. The inventors therefore tested for genetic interactions between *dBRDW3* and JAK/STAT signalling by crossing the *dBRDW3⁰⁵⁸⁴²* allele to *GMR-updΔ3'* (Bach, E. A., Vincent, S., Zeidler, M. P. & Perrimon, N., Genetics 165, 1149-66 (2003)). The *GMR-upd*□*3*' transgene ectopically misexpress Upd during eye development resulting in cellular overproliferation and an enlarged adult eye (Fig. 3b (left)). Furthermore, removal of one copy of *stat92E* significantly suppresses eye overgrowth (Fig. 3b (middle)) due to a reduction in the potency of JAK/STAT signalling (Bach, E. A., Vincent, S., Zeidler, M. P. & Perrimon, N., Genetics 165, 1149-66 (2003)). Removal of a single copy of *dBRDW3* was also able to suppress the *GMR-updΔ3'* phenotype (Fig. 3b (right)) as expected for a positive regulator of JAK/STAT signalling. In addition, a chromosomal deficiency removing the region has also been independently identified as a suppressor of *GMR-updΔ3'* (Bach, E. A., Vincent, S., Zeidler, M. P. & Perrimon, N., Genetics 165, 1149-66 (2003)).

One phenotypic consequence of constitutive JAK/STAT activation caused by the gain-of-function JAK allele *hop^{Tuml}* is the overproliferation of haemocytes and the frequent formation of melanotic tumours, a phenotype previously described as a *Drosophila* model for leukaemia (Luo, H., Hanratty, W. P. & Dearolf, C. R., Embo J 14, 1412-20 (1995); Harrison, D. A., Binari, R., Nahreini, T. S., Gilman, M. & Perrimon, N., Embo J 14, 2857-65 (1995)). The inventors found that the removal of one copy of *dBRWD3* is sufficient to reduce the size and the frequency of *hop^{Tuml}* induced melanotic tumours (Fig. 3c and Table 3). Moreover, homozygous escapers of a putative hypomorphic allele of *dBRWD3,* generated by excision of the original P-element, frequently develop ectopic wing vein material (Fig. 3d) reminiscent of the weak loss-of-function *stat92E^{HJ}* allele (Yan, R., Luo, H., Darnell, J. E., Jr. & Dearolf, C. R., Proc Natl Acad Sci U S A 93, 5842-7 (1996)). Taken together, these experiments suggest a role for *dBRWD3* in JAK/STAT signalling.

As a second example the inventors analysed the *ptp61F* gene which encodes a protein tyrosine phosphatase. dsRNA knocking down all mRNA splice forms transcribed from this locus leads to an increase in JAK/STAT signalling activity. The inventors performed epistasis analysis in which the inventors removed known pathway components and tested for the effect of simultaneously targeting *ptp61F.* Double RNAi against *ptp61F* together with *lacZ, dome* or *hop* results in pathway stimulation (Fig. 4a). However, simultaneous removal of *ptp61F* and *stat92E* is sufficient to prevent signalling (Fig 4a). Loss of this phosphatase therefore results in the stimulation of STAT92E activity even in the absence of upstream components indicating that Ptp61F negatively regulates the pathway downstream of JAK. The inventors next asked whether Ptp61F also interferes with JAK/STAT signalling *in vivo* by using the *cg-Gal4* driver to misexpress *ptp61F* in blood cells of *hop^{Tuml}* mutants. Misexpression of Ptp61 Fc in a *hop^{Tuml}* mutant background resulted in a suppression of melanotic tumour formation with the average frequency of large tumours reduced by approximately 4 fold, an effect also observed following the misexpression of the known negative regulator dPIAS (Betz, A., Lampen, N., Martinek, S., Young, M. W. & Darnell, J. E., Jr., Proc Natl Acad Sci U S A 98, 9563-8 (2001)) (Fig. 4b and Table 3). Alternative splicing of *ptp61F* leads to nuclear and cytoplasmic protein forms which both contain the same phosphatase domain (McLaughlin, S. & Dixon, J. E., J Biol Chem 268, 6839-42 (1993)). However, the tumour suppressor phenotype is only observed with nuclear Ptp61Fc (Fig. 4c), an effect that is reproduced by over-expression of the nuclear localised protein in cell culture (Fig. 4c). These results are consistent with our identification of *ptp61F* as a negative regulator of pathway activity and suggest that it may function by targeting phosphorylated, nuclear localised STAT92E for deactivation.

Aberrant JAK/STAT signalling has been implicated in multiple human malignancies and its components have been proposed as molecular targets for the development of therapeutic compounds (O'Shea, J. J., Pesu, M., Borie, D. C. & Changelian, P. S., Nat Rev Drug Discov 3, 555-64 (2004)). The genome-wide screen presented here identified known and previously unknown genes and the inventors have characterised their likely level of interaction with defined pathway components using cell-based epistasis analysis. Of the 89 JAK/STAT modifiers identified, many have human homologues that remain to be characterised. The inventors have here performed an analysis of two examples *in vivo* and demonstrate their roles in regulating the pathway during development and tumour genesis in *Drosophila.* One of these is a homologue of human *BRWD3,* a gene recently identified at the break-point of a translocation isolated from multiple B-CLL patients (Kalla, C. et al., Genes Chromosomes Cancer 42, 128-43 (2005)). Given our functional analysis of *dBRWD3* and the known roles for JAK/STAT signalling during normal haematopoiesis, it is possible that a breakdown in *BRWD3* mediated STAT regulation may represent a key molecular mechanism leading to the development of B-CLL. Thus, comprehensive reverse genetic surveys for signalling pathway components using *Drosophila* as a model organism represent a potentially powerful approach with which insights relevant to human disease can be obtained.

**Table 1: JAK/STAT phenotypes by RNAI**

| Gene name | dsRNA ID | *z*-score [6x2xDraf-luc] | *z*-score [4xSOCS-luc] | Functional group assignment (based on GO end Interpro evidence) | Interpro 8.0 evidence | GO Evidence | SEQ ID NO. |
|---|---|---|---|---|---|---|---|
| Positive Regulators | | | | | | | |
| Art2 | HFA00627 | -2,9 | -3,2 | Protein modifying enzymes / Metabolism | IPR000051 | GO:0016274; protein-arginine N-methyltransterase activity | SEQ ID NO. 175 |
| asf1 | HFA11324 | -2,3 | -2,5 | Others | IPR008967 | GO:0003682; chromatin binding | SEO ID NO. 176 |
| bin3 | HFA04919 | -3,1 | -3,3 | Unknown | IPR000051 | na; na | SEQ ID NO. 177 |
| CG10007 | HFA14173 | -3,2 | -2,9 | Unknown | nolPR | na; na | SEQ ID NO. 178 |
| CG10730 | HFA02102 | -2,1 | -2,3 | Unknown | IPR004245 | na; na | SEQ ID NO. 179 |
| CG10960 | HFA09807 | -2,0 | -2,1 | Protein modifying enzymes / Metabolism | IPR005829 | GO:0005355; glucose transporter activity | SEO ID NO. 180 |
| CG11307 | HFA11648 | -2,3 | -2,4 | Unknown | nolPR | GO:0016757; translerase activity | SEQ ID NO. 181 |
| CG11696 | HFA19417 | -2,0 | -2,3 | Transcription regulators | IPR007087 | GO:0003677; DNA binding | SEQ ID NO. 182 |
| CG12213 | HFA14478 | -3,3 | -3,2 | Unknown | IPR009053 | na;na | SEO ID NO. 183 |
| CG12460 | HFA20970 | -3,3 | -3,4 | Transcription regulators | IPR000504 | GO:0030528; transcription regulator activity | SEQ ID NO. 184 |
| CG12479 | HFA19459 | -2,3 | -2,4 | Unknown | IPR007512 | na;na | SEQ ID NO. 185 |
| CG13243 | HFA01920 | -2,7 | -2,6 | Unknown | IPR003117 | na;na | SEQ ID NO. 186 |
| CG13473 | HFA10017 | -2,4 | -2,1 | Cytoskeleton and Transport | IPR006662 | GO:0005489; electron transporter activity | SEQ ID NO. 187 |
| CG14434 | HFA17927 | -2,0 | -2,3 | Unknown | IPR008173 | na; na | SEQ ID NO. 188 |
| CG15306 | HFA17993 | -3,3 | -3,1 | Signal transduction | IPR001715 | GO:0005102; receptor binding | SEQ ID NO. 189 |
| CG15418 | HFA00432 | -2,1 | -2,1 | Protein modifying enzymes / Metabolism | IPR002223 | GO:0004866; endopeptidase inhibitor activity | SEQ ID NO. 190 |
| CG15434 | HFA00449 | -2,5 | -2,9 | Protein modifying enzymes / Metabolism | IPR007741 | GO:0003954; NADH dehydrogenase activity | SEQ ID NO. 191 |
| CG15555 | HFA15093 | -2,3 | -2,6 | Others | IPR001873 | GO:0015268; alpha-type channel activity | SEQ ID NO. 192 |
| CG15784 | HFA18090 | -2,4 | -2,6 | Unknown | IPR009072 | na; na | SEQ ID NO. 193 |
| CG16903 | HFA18561 | -2,8 | -2,8 | Transcription regulators | IPR011028 | GO:0016251; general RNA polymerase 11 transcription lector activity | SEQ ID NO. 194 |
| CG17179 | HFA10258 | -2,1 | -2,8 | Unknown | IPR001660 | na; na | SEQ ID NO. 195 |
| CG18160 | HFA21006 | -3,1 | -2,4 | Unknown | nolPR | na;na | SEQ ID NO. 196 |
| CG30069 | HFA06272 | -2,9 | -2,2 | Protein modifying enzymes / Metabolism | nolPR | GO:0016491; oxidoreductase activity | SEQ ID NO. 197 |
| CG3058 | HFA00563 | -3,4 | -3,5 | Cytoskeleton and Transport | IPR006663 | GO:0005489; electron transporter activity | SEQ ID NO. 198 |
| CG31005 | HFA15507 | -2,3 | -3,0 | Protein modifying enzymes / Metabolism | IPR000092 | GO:0000010; trans-hexaprenyltranstransferase activity | SEQ ID NO. 199 |
| CG31132 | HFA16032 | -2,8 | -3,5 | Unknown | IPR001487 | na; na | SEQ ID NO. 200 |
| CG31132 | HFA15369 | -2,3 | -3,6 | Unknown | IPR001487 | na; na | SEQ ID NO. 201 |
| CG31358 | HFA15235 | -2,0 | -2,2 | Cytoskeleton and Transport | IPR001972 | GO:0005200; structural constituent of cytoskeleton | SEQ ID NO. 202 |
| CG31694 | HFA00415 | -2,8 | -2,7 | Signal transduction | IPR006921 | GO:0005102; receptor binding | SEQ ID NO. 203 |
| CG32406 | HFA09966 | -2,1 | -2,2 | Signal transduction | IPR000980 | na; na | SEQ ID NO. 204 |
| CG32573 | HFA19906 | -3,1 | -2,9 | Unknown | IPR000719 | na; na | SEQ ID NO. 205 |
| CG3281 | HFA15470 | -3,1 | -3,0 | Transcription regulators | IPR007087 | GO:0030528; transcription regulator activity | SEQ ID NO. 206 |
| CG3819 | HFA10376 | -2,3 | -2,3 | Unknown | IPR001604 | na; na | SEQ ID NO. 207 |
| CG4022 | HFA10395 | -3,4 | -3,7 | Unknown | nolPR | na;na | SEQ ID NO. 208 |
| CG40351 | HFA20930 | -2,6 | -2,7 | Transcription regulators | IPR001214 | GO:0030528; transcription regulator activity | SEQ ID NO. 209 |
| CG4349 | HFA19892 | -4,1 | -2,1 | Others | IPR009040 | GO:0008199; ferric iron binding | SEQ ID NO. 210 |
| CG4446 | HFA10420 | -2,7 | -2,7 | Protein modlfying enzymes / Metabolism | IPR004625 | GO:0008478; pyridoxal kinase activity | SEQ ID NO. 211 |
| CG4653 | HFA19909 | -3,2 | -3,0 | Protein modifying enzymes / Metabolism | IPR001254 | GO:0004263; chymotrypsin activity | SEQ ID NO. 212 |
| CG4781 | HFA04488 | -2,5 | -2,5 | Unknown | IPR003591 | na; na | SEQ ID NO. 213 |
| CG6422 | HFA16036 | -3,3 | -3,2 | Unknown | IPR007275 | na; na | SEQ ID NO. 214 |
| CG6434 | HFA10635 | -2,8 | -2,8 | Unknown | IPR001680 | na; na | SEQ ID NO. 215 |
| CG6946 | HFA16145 | -2,3 | -2,9 | RNA processing and Translation | IPR000504 | GO:0003723; RNA binding | SEO ID NO. 216 |
| CG7635 | HFA20054 | -2,9 | -2,8 | Cytoskeleton and Transport | IPR001972 | GO:0005200; structural constituent of cytoskeleton | SEO ID NO. 217 |
| CG8108 | HFA09675 | -2,7 | -2,7 | Transcription regulators | IPR007087 | GO:0003676; nucleic acid binding | SEO ID NO. 218 |
| CG9086 | HFA20148 | -2,8 | -2,9 | Signal transduction | IPR009030 | GO:0005057; receptor signaling protein activity | SEO ID NO. 219 |
| Ckllalpha | HFA11946 | -2,1 | -2,5 | Signal transduction | IPR000719 | GO:0004702; receptor signaling protein serine/threonine kinase activity | SEQ ID NO. 220 |
| Ckllbeta | HFA20230 | -2,7 | -2,6 | Signal transduction | IPR000704 | GO:0004702; receptor signaling protein serine/threonine kinase activity | SEO ID NO. 221 |
| comm3 | HFA09995 | -2,2 | -2,2 | Unknown | nolPR | na; na | SEQ ID NO. 222 |
| CtBP | HFA16617 | -2,9 | -2,8 | Transcription regulators | IPR006139 | GO:0003714; transcription corepressor activity | SEQ ID NO. 223 |
| dome | HFA19583 | -6,2 | -4,9 | Signal transduction | IPR000194 | GO:0004907; interleukin receptor activity | SEQ ID NO. 224 |
| elF·4B | HFA20983 | -3,2 | -3,0 | RNA processing and Translation | IPR000504 | GO:0003723; RNA binding | SEQ ID NO. 225 |
| HDC01676 | HFA01091 | -2,3 | -2,6 | Unknown | IPR006202 | na;na | SEQ ID NO. 226 |
| HDC11198 | HFA11427 | -2,3 | -2,2 | Unknown | nolPR | na; na | SEQ ID NO. 227 |
| hop | HFA20340 | -5,7 | -4,1 | Signal transduction | IPR001245 | GO:0004718; Janus kinase activity | SEQ ID NO. 228 |
| Ipk2 | HFA00357 | -2,6 | -4,0 | Signal transduction | IPR005522 | GO:0050516; inosltol-polyphosphate multikinase activity | SEQ ID NO. 229 |
| jbug | HFA04167 | -2,7 | -3,2 | Cytoskelaton and Transport | IPR001298 | GO:0005200; structural constituent of cytoskeleton | SEQ ID NO. 230 |
| kn | HFA07637 | -2,4 | -2,4 | Transcription regulators | IPR003523 | GO:0030528; transcription regulator activity | SEQ ID NO. 231 |
| l(1)G0084 | HFA19450 | -2,1 | -2,1 | Transcription regulators | IPR001965 | GO:0003677; DNA binding | SEQ ID NO. 232 |
| larp | HFA16984 | -2,5 | -2,4 | Unknown | IPR006630 | na; na | SEQ ID NO. 233 |
| mask | HFA15370 | -2,3 | -2,7 | Signal transduction | IPR002110 | GO:0005102; receptor binding | SEQ ID NO. 234 |
| mst | HFA20582 | -2,2 | -2,6 | Unknown | nolPR | na; na | SEQ ID NO. 235 |
| nonA | HFA20357 | -3,0 | -3,3 | RNA processing and Translation | IPR000504 | GO:0030528; transcription regulator activity | SEQ ID NO. 236 |
| Obp93a | HFA15220 | -2,4 | -2,9 | Cytoskeleton and Transport | IPR006170 | GO:0005549; odorant binding | SEQ ID NO. 237 |
| Rrp1 | HFA00784 | -4,3 | -4,3 | Others | IPR000097 | GO:0004520; endodeoxyribonuclease activity | SEQ ID NO. 238 |
| sol | HFA20587 | -2,5 | -3,0 | Others | IPR001876 | GO:0005516; calmodulin binding | SEQ ID NO. 239 |
| Stat92E | HFA16870 | -5,0 | -5,2 | Signal transduction | IPR001217 | GO:0004871; signal transducer activity | SEQ ID NO. 240 |
| Taf2 | HFA11298 | -2,7 | -2,9 | Transcription regulators | IPR002052 | GO.0016251; general RNA polymerase II transcription factor activity | SEQ ID NO. 241 |

| Negative regulators | | | | | | | |
|---|---|---|---|---|---|---|---|
| bon | HFA16914 | 5,6 | 4,8 | Protein modifying enzymes / Metabolism | IPR001841 | GO:0004842; ubiquitin-protein ligase activity | SEQ ID NO. 242 |
| Caf1 | HFA16596 | 3,0 | 2,6 | Protein modifying enzymes / Metabolism | IPR001680 | GO:0035035; histone acetyltransferase binding | SEQ ID NO. 243 |
| CG10077 | HFA09691 | 2,6 | 4,0 | RNA processing and Translation | IPR001410 | GO:0003724; RNA hellcase activity | SEQ ID NO. 244 |
| CG11400 | HFA06070 | 2,6 | 2,2 | Unknown | nolPR | na; na | SEQ ID NO. 245 |
| CG11501 | HFA14317 | 3,7 | 3,1 | Unknown | nolPR | na; na | SEQ ID NO. 246 |
| CG13499 | HFA04144 | 2,5 | 3,1 | Unknown | nolPR | na; na | SEQ ID NO. 247 |
| CG14247 | HFA14742 | 3,2 | 3,4 | Unknown | IPR002557 | na; na | SEQ ID NO. 248 |
| CG15706 | HFA06577 | 2,2 | 2,1 | Unknown | IPR011701 | na; na | SEQ ID NO. 249 |
| CG16975 | HFA02552 | 2,7 | 2,7 | Transcription regulators | IPR001660 | GO:0030528; transcription regulator activity | SEQ ID NO. 250 |
| CG17492 | HFA02623 | 2,5 | 2,1 | Protein modifying enzymes / Metabolism | IPR001841 | GO:0004842; ubiquitin-protein ligase activity | SEQ ID NO. 251 |
| CG16112 | HFA15304 | 2,1 | 2,1 | Unknown | IPR001829 | na; na | SEQ ID NO. 252 |
| CG30122 | HFA06935 | 3,3 | 2,8 | Transcription regulators | IPR003034 | GO:0003677; DNA binding | SEQ ID NO. 253 |
| CG4907 | HFA15673 | 3,3 | 3,5 | Unknown | IPR007070 | na;na | SEQ ID NO. 254 |
| dre4 | HFA08714 | 2,6 | 2,5 | Transcription regulators | IPR000994 | GO:0003712; transcription cotactor activity | SEQ ID NO. 255 |
| enok | HFA04096 | 3,0 | 3,0 | Transcription regulators | IPR001965 | GO:0030528; transcription regulator activity | SEQ ID NO. 256 |
| lig | HFA07247 | 2,2 | 2,1 | Unknown | IPR009060 | na;na | SEQ ID NO. 257 |
| Nup154 | HFA03364 | 2,9 | 2,9 | Cytoskeleton and Transport | IPR011045 | GO:0005487; nucleocytoplasmic transporter activity | SEO ID NO. 258 |
| par-1 | HFA07660 | 4,4 | 4,2 | Signal transduction | IPR000719 | GO:0004674; protein serine/threonine kinase activity | SEO ID NO. 259 |
| Ppielpha-96A | HFA16795 | 3,0 | 3,8 | Signal transduction | IPR006186 | GO:0004722; protein serine/threonine phosphatase activity | SEO ID NO. 260 |
| PP2A-B' | HFA16344 | 2,6 | 2,5 | Signal transduction | IPR002554 | GO:0008601; protein phosphatase type 2A regulator activity | SEQ ID NO. 261 |
| Ptp61F | HFA08683 | 5,9 | 8,1 | Signal transduction | IPR000863 | GO:0004725; protein tyrosine phosphatase activity | SEQ ID NO. 262 |
| Rab5 | HFA00777 | 2,1 | 2,1 | Signal transduction | IPR001806 | GO:0005525: GTP binding | SEQ ID NO. 263 |
| Socs36E | HFA02455 | 3,2 | 2,3 | Signal transduction | IPR000980 | GO:0007259; JAK·STAT cascade | SEO ID NO. 264 |
| TSG101 | HFA11098 | 3,1 | 3,4 | Protein modifying enzymes / Metabolism | IPR001440 | GO:0004842; ubiquitin-proteln ligase activity | SEO ID NO. 265 |

| | | | | | | | |
|---|---|---|---|---|---|---|---|
| InterPro Evidence was obtained from: Mulder et al. (2005). InterPro, progress and status In 2005. GO Evidence was obtained from: R.A. Drysdale, M.A. Crosby and The FlyBase Consortium (2005). FlyBase: genes and gene models. Nucleic Adds Research 33:D390-D395. http://flybase.org/ All 384-well screening plates contained dsRNAs against known JAK/STAT pathway components. Controls for the 57 screening plates were *stat92E* RNAI (identified 55 times), *hop* RNAI (identified 37 times), dome RNAI (identified 55 times) and *socs36E* RNAI (Identified 45 times) An Interactive table with links to the Interpro records Is available at http://www.dkfz.de/slgnallng/]ak-pathway/ | | | | | | | |

**Table 2: Functional groups classified by InterPro prediction and GO.**

| Functional Group^{†} | N* |
|---|---|
| Signalling factors | 17 |
| Transcription factors | 14 |
| Protein modification and Metabolism | 12 |
| Cytoskeleton and Transport | 7 |
| All others | 9 |
| Predicted proteins classified as part of a | 59 |
| Predicted proteins without classification | 31 |

| | |
|---|---|
| Queries were performed with InterPro 8.0 † InterPro and GO results classified into one of functionally related groups. See Table 1 for complete list of genes, specific IPR domains and GO assigned within each group. * Number of proteins identified with InterPro domains and/or GO found in 90 translated gene sequences. | |

**Supplementary Table 3: Genetic interactions with hop^{Tuml(1)}**

| **Exp** | **Genotype** | **Insert/Allele** | **Tumours (%)** | | | **n** | **z-score** | |
|---|---|---|---|---|---|---|---|---|
| | | | **None** | **Small** | **Large** | | | |
| I | *y,w,hop^{Tuml}*/*+;* +/+ | OreR | **31.0** | **50.6** | **18.4** | 358 | -0.4 | ^{(*)} |
| II | *y,w,hop^{Tuml}*/*+;* +/+ | OreR | **31.0** | **43.8** | **25.2** | 445 | -0.4 | ^{(*)} |
| II | *y,w,hop^{Tuml}*/*+;* +/+ | *w1118* | **23.9** | **31.2** | **44.9** | 356 | 0.6 | ^{(*)} |
| | | | | | | | | |
| II | *y,w,hop^{Tuml}*/*+; STAT92E*/*+* | *397* | **67.5** | **21.5** | **11.0** | 228 | -5.3 | (2) |
| I | *y,w,hop^{Tuml}*/*+ ; STAT92E*/*+* | *06346* | **68.6** | **26.1** | **5.3** | 283 | -5.4 | (3) |
| II | *y,w,hop^{Tuml}*/*+* ; *STAT92E*/*+* | *06346* | **64.2** | **26.6** | **9.2** | 282 | -4.9 | (3) |
| | | | | | | | | |
| II | *y,w,hop^{Tuml}*/*+; dBRWD3*/*+* | *05842* | **56.6** | **24.4** | **19.0** | 221 | -3.8 | |
| I | *y,w,hop^{Tuml}*/*+;cg-Gal4*/*UAS-EGFP* | 5a.2 | **19.9** | **35.1** | **45.0** | 151 | 1.2 | ^{(*)} |
| II | *y,w,hop^{Tuml}*/*+;cg-Gal4*/*UAS-EGFP* | 6a.3 | **41.0** | **33.3** | **25.7** | 451 | -1.7 | ^{(*)} |
| II | *y,w,hop^{Tuml}*/*+;cg-Gal4*/*UAS-lacZ* | BG4-1-2 | **25.8** | **26.4** | **47.8** | 341 | 0.4 | ^{(*)} |
| | | | | | | | | |
| II | *y,w,hop^{Tuml}*/*+;cg-Gal4*/*UAS-ptp61Fa* | 1b.2 | **46.5** | **27.7** | **25.7** | 101 | -2.5 | |
| I | *y,w,hop^{Tuml}*/*+;cg-Gal4*/*UAS-ptp61Fa* | 1b.2 | **46.5** | **29.1** | **24.3** | 230 | -2.5 | |
| I | *y,w,hop^{Tuml}*/*+;cg-Gal4*/*UAS-ptp61Fa* | 1a.3 | **22.6** | **28.8** | **48.6** | 177 | 0.8 | |
| II | *y,w,hop^{Tuml}*/*+;cg-Gal4*/*UAS-ptp61Fa* | 1a.3 | **19.6** | **24.4** | **56.0** | 168 | 1.2 | |
| II | *y,w,hop^{Tuml}*/*+;cg-Gal4*/*UAS-ptp61Fa* | 3a.3 | **35.8** | **28.5** | **35.8** | 165 | -1.0 | |
| I | *y,w,hop^{Tuml}*/*+;cg-Gal4*/*UAS-ptp61Fa* | 7a.3 | **16.4** | **36.1** | **47.5** | 61 | 1.6 | |
| | | | | | | | | |
| II | *y,w,hop^{Tuml}*/*+;cg-Gal4*/*UAS-ptp61Fc* | 1a.1 | **68.2** | **21.4** | **10.4** | 280 | -5.4 | |
| II | *y,w,hop^{Tuml}*/*+;cg-Gal4*/*UAS-ptp61Fc* | 2a.4 | **56.1** | **30.6** | **13.3** | 255 | -3.8 | |
| I | *y,w,hop^{Tuml}*/*+;cg-Gal4*/*UAS-ptp61Fc* | 2a.4 | **52.3** | **40.7** | **7.0** | 344 | -3.2 | |
| I | *y,w,hop^{Tuml}*/*+;cg-Gal4*/*UAS-ptp61Fc* | 2b.3 | **59.4** | **33.8** | **6.8** | 234 | -4.2 | |
| II | *y,w,hop^{Tuml}*/*+;cg-Gal4*/*UAS-ptp61Fc* | 2b.3 | **63.3** | **29.3** | **7.3** | 300 | -4.7 | |
| | | | | | | | | |
| II | *y,w,hop^{Tuml}*/*+;cg-Gal4*/*UAS-dPIAS-GFP* | 26b.3 | **67.0** | **27.4** | **5.7** | 106 | -5.2 | (4) |
| I | *y,w,hop^{Tuml}*/*+;cg-Gal4*/*UAS-dPIAS-GFP* | 26b.3 | **63.1** | **33.6** | **3.3** | 122 | -4.7 | (4) |

| | | | | | | | | |
|---|---|---|---|---|---|---|---|---|
| Values shown represent percentage of 0-24hr old female flies containing no, small or large tumours visible in abdomen or thorax. Table shows results from two independent experiments (first column) undertaken under identical conditions. (*) 'wild type' results used to calculate z-scores | | | | | | | | |

### References:

(1) Hanratty, W. P. & Dearolf, C. R. The Drosophila Tumorous-lethal hematopoietic oncogene is a dominant mutation in the hopscotch locus. Mol Gen Genet 238, 33-7 (1993).
(2) Silver, D. L. & Montell, D. J. Paracrine signaling through the JAK/STAT pathway activates invasive behavior of ovarian epithelial cells in Drosophila. Cell 107, 831-841 (2001).
(3) Hou, X. S., Melnick, M. B. & Perrimon, N. Marelle acts downstream of the Drosophila HOP/JAK kinase and encodes a protein similar to the mammalian STATs. Cell 84, 411-9 (1996).
(4) Betz, A., Lampen, N, Martinek, S., Young, M. W. & Darnell, J. E. Jr. A Drosophila PIAS homologue negatively regulates stat92E. Proc Natl Acad Sci USA 98, 9563-8 (2001).

**Table 4: Sequence and cytological Information**

| **dsRNA ID** | **Amplicon primer 1A** | **Amplicon primer 2** | **No of efficient sIRNAsB** | **Target gene (Symbol)** | **Cytological location** | **SEQ ID NOs.** |
|---|---|---|---|---|---|---|
| HFA00627 | TGC CTG TIT TCT GGA AAT ATG | CTC GCT GGG TTT CAT GGT | 51/496 | Art2 | 24E1 | SEQ ID NOs. 266/267 |
| HFA11324 | TCG AAC TCA CGT TCG AGT | ATC ATC TTC GGG ATG GAT AAC | 61/489 | asf1 | 76B9 | SEQ ID NOs. 268/269 |
| HFA04919 | GAG ATA CCC CGT GAT GAC A | CTT GGG AAT ACG CAC AAA GA | 87/487 | bin3 | 42A13··14 | SEQ ID NOs. 270/271 |
| HFA16914 | AGG TGC TGG TGG AAA AGA A | ACC CGT CAC CCG GAA AG | 60/496 | bon | 92F2··3 | SEQ ID NOs. 272/273 |
| HFA16596 | TAT TTG CTG TCA GCC TCT G | TGG TCC GTC CTC AGC ATC | 81/496 | Caf1 | 88E3 | SEQ ID NOs. 274/275 |
| HFA14173 | CGC CCT GAT CTT TGT GGG | GGA CGA GTA CAT CGC AAT G | 139/494 | CG10007 | 87A4 | SEQ ID NOs, 276/277 |
| HFA09691 | GCA CCA CCT CGT TGA AGA | GGG CAG CCA CAT CGG T | 72/484 | CG10077 | 65D3··4 | SEQ ID NOs. 278/279 |
| HFA02102 | GAA CTT CAT TTG GAA GCG TTT | CTT GCG CCG GAA CCA G | 103/497 | CG10730 | 38B2 | SEQ ID NOs. 280/281 |
| HFA09807 | GCC GCC GGT ACC GTC | AAG TAG GTG GGC GAT TCC | 63/495 | CG10960 | 69E5··6 | SEQ ID NOs. 282/283 |
| HFA11648 | CCG TGG CCA CAG GAA CA | CAG TCC TGT TCA TGT GGA AAT | 51/242 | CG11307 | 78E1 | SEQ ID NOs. 284/285 |
| HFA06070 | TTG TCT GGC TGT GTC TGT C | GAC AAT CCT TGG CCC AAT AAC | 85/459 | CG11400 | 54A1 | SEQ ID NOs. 286/287 |
| HFA14317 | ATG GCA TCC CCA GTA GTC A | GTG ACT TTG ATG ATC TGG ATT C | 28/312 | CG11501 | 99B1 | SEQ ID NOs. 288/289 |
| HFA19417 | GCC GAC GAA CAG CCA AA | TCG CAC ACC TCG GGA C | 64/486 | CG11696 | 10C7 | SEQ ID NOs. 290/291 |
| HFA14478 | TAA CGG TGA CGG AAC CCA | CCG AAT CCT CGA TGG GTT | 78/498 | CG12213 | 87A3 | SEQ ID NOs. 292/293 |
| HFA20970 | GCC AAA ATC AAG CGA ATC AG | CTT AAT TGC CTG CAC CTC C | 50/114 | CG12460' | heterochromatin | SEQ ID NOs. 294/295 |
| HFA19459 | ATC GGC TGC GTG AGA AC | TTC GTT GGC CAA ACT TTA CA | 19/181 | CG12479 | 12E2 | SEQ ID NOs. 296/297 |
| HFA01920 | GAT TGG ACG CTT CTG TIT GA | GTT GAA ACA TTG CTG GGT GA | 112/494 | CG13243 | 3SD4··5 | SEQ ID NOs. 298/299 |
| HFA10017 | TGG CTG CCA TGC AGA AG | CCA ATT TCG GCA CGG TAG | 73/391 | CG13473 | 70F3 | SEQ ID NOs. 300/301 |
| HFA04144 | AGT GGC AGC GGA GGT G | GCC CTC GCA GTG GGT T | 19/256 | CG13499 | 58B1 | SEQ ID NOs. 302/303 |
| HFA14742 | AAA ATA AAT GGA GTA ACT TCC CC | TAC GCC TCG CAC TCC A | 34/497 | CG14247 | 97D1 | SEQ ID NOs. 304/305 |
| HFA17927 | CGC AAT GTG GAG GTG AAG | ATC GAA ATA CGA GCC GAT C | 52/490 | CG14434 | 6D7 | SEQ ID NOs. 306/307 |
| HFA17993 | TTC GAG GGC CCA CAA TGT | TGG CAA GTC GCA ACT TTA C | 122/475 | CG15306 | 9B7 | SEQ ID NOs. 308/309 |
| HFA00432 | CAA AGG CAC CTG GTT TGT G | CAG TAG CGC AGA CGT TG | 19/143 | CG15418 | 24A2 | SEQ ID NOs. 310/311 |
| HFA00449 | GGT ATT ACT CTG TTC CGA TTG | CTT CCA GGT TTT TGT GTA TGT C | 30/217 | CG15434 | 24F3 | SEQ ID NOs. 312/313 |
| HFA15093 | GGC AAA GAT CCC AAG CAG | GTT GAA GGT GCA GCA GAA G | 58/283 | CG15555 | 100B9 | SEQ ID NOs. 314/315 |
| HFA06577 | CAG CCA TCG ATT GGA ACA G | CTC CAA GTG CCA GAA CAT AAA | 77/477 | CG15706 | 52F11 | SEQ ID NOs. 316/317 |
| HFA18090 | GGC CAC AAG CAT GGT CG | CCT TGC CCT TGC ACT TCT | 51/500 | CG15784 | 4F10 | SEQ ID NOs. 318/319 |
| HFA18561 | TCG CCC ATG GTG CTA GA | CGA TCC ACG GTG ATT ACA G | 72/477 | CG16903 | 2C10 | SEQ ID NOs. 320/321 |
| HFA02552 | CAG ACT CCT ACC TCG TTT TG | AAC ATG CGC TCC AGA TAG T | 54/495 | CG16975 | 34A7--8 | SEQ ID NOs. 322/323 |
| HFA10258 | GCC AAG AGA CGG AGA AGA | TAC GGA TGC TGG TTG ATG T | 3/155 | CG17179' | U | SEQ ID NOs. 324/325 |
| HFA02623 | CCC AGG GCC ATT TGG ATT T | TCC TTT AAG CGC TGC ATG | 71/486 | CG17492 | 37B10--11 | SEQ ID NOs. 326/327 |
| HFA15304 | GGG CAT GCC GTC ATT ACA | CGG CGA TAT TTG CTG GTC | 78/475 | CG18112 | 99C2 | SEQ ID NOs. 328/329 |
| HFA21006 | GTG GCG CAC CGG AAA G | GAT GAA CTT CAT TGT TGT TGA AA | 50/114 | CG18160' | U | SEQ ID NOs. 330/331 |
| HFA06272 | TGA CGA AGC ATA TAC AAG GAT A | TGG GTT TTT CTG GTG AAA CAA | 111/489 | CG30069 | 50E2--3 | SEQ ID NOs. 332/333 |
| HFA06935 | GTT TGC ATC GGC CAA ACC | GTG TCA GAG AAA TTC ACT AAG TA | 62/463 | CG30122 | 55E3 | SEQ ID NOs. 334/335 |
| HFA00563 | AAT ACG TTT CGG TCA CGA TT | GTA TCT GTA CTT GGT AGA GTA GT | 69/326 | CG3058 | 24F1 | SEQ ID NOs. 336/337 |
| HFA15507 | CCC CGA GCT GAA TCC CA | CTT CAT GCG GTT GAT GAC TA | 8/197 | CG31005 | 100B8 | SEQ ID NOs. 338/339 |
| HFA15369 | CGT AAG TGC TAG TTC CTC TG | TGC CGA GCG TCC CTT T | 34/488 | CG31132 | 95F12--13 | SEQ ID NOs. 340/341 |
| HFA16032 | CCC ACG GAG CTG TTC TTT | AAA CGA CTA CCC AGG ACA TT | 63/495 | CG31132 | 95F12--13 | SEQ ID NOs. 342/343 |
| HFA15235 | AGG CAT CTG CAG ATT CTC T | GAG GAA TGG GAA TGG ATG AAG | 112/488 | CG31358 | 87A5 | SEQ ID NOs. 344/345 |
| HFA00415 | GTC ATG GGT CCC GGG ATG | TCG CTT GTC ACG ATT CTT T | 28/159 | CG31694 | 23B7--8 | SEQ ID NOs. 346/347 |
| HFA09966 | CCG CCA CAA TGA TAA CCA AC | CGC GTG CGT GAA GAG T | 68/477 | CG32406 | 65A2--3 | SEQ ID NOs. 348/349 |
| HFA19906 | ATC TGT TGA ACG CCG AGG | GGT ATC GGT GAA GTT CTT CTC | 39/495 | CG32573 | 14F5 | SEQ ID NOs. 350/351 |
| HFA15470 | TTG TCG CGA CCT TCC CA | ACT TCT TGG AGC AGA TCT TG | 66/500 | CG3281 | 87A3 | SEQ ID NOs. 352/353 |
| HFA10378 | CGG ACA CCG GCT ATG TG | ATG TTC TTG GCC GAG TCA A | 70/482 | CG3819 | 75E6 | SEO ID NOs. 354/355 |
| HFA10395 | TAC TCA AGG ATC GCG ATA TC | GGC TGG GTG TGG GAG TG | 53/484 | CG4022 | 67B4--5 | SEQ ID NOs. 356/357 |
| HFA20930 | GCA GGA CGT TCG GAA TAT C | TCC CAT TAC AGA CTT TTG ATT G | 199/540 | CG40351 | U | SEQ ID NOs. 358/359 |
| HFA19892 | GGC GCC ACA TGT GCA TG | GCC GCT GCC CAT ATA CTT | 97/480 | CG4349 | 11D11 | SEQ ID NOs. 360/361 |
| HFA10420 | TGT GGC TGT CGC TTA TCT T | AAA AAT ATA CAG CCG TTT CCT T | 56/481 | CG4446 | 67B2 | SEQ ID NOs. 362/363 |
| HFA19909 | ACC CAG CTA AAT CCT ACA ATG | ACT CCA GAT GCT GGG TCA | 55/496 | CG4653 | 15A3 | SEQ ID NOs. 364/365 |
| HFA04488 | TTG ACG GAT TGC CAC ATC T | GCC TCC GCG TCC AAG T | 75/482 | CG4781 | 60D10 | SEQ ID NOs. 366/367 |
| HFA15673 | TGG GCT CGG CAG AGA TA | CAA GTA GAG GAG CCC GAT | 105/492 | CG4907 | 94C2 | SEQ ID NOs. 368/369 |
| HFA16036 | TCT TTG TCA TCA AAT CGT ACT C | CAT CGG GCC CAT GCA TT | 102/487 | CG6422 | 96B17 | SEQ ID NOs. 370/371 |
| HFA10635 | TTG AAC ATC GTG GCT TCT TT | CCT CGC AAA CTC GAT GC | 39/148 | CG6434 | 7784 | SEQ ID NOs. 372/373 |
| HFA16145 | CAA CAA CAT GCT GGG CTT C | CGA AGT TCG AGC CGA CA | 118/468 | CG6946 | 86F8--9 | SEQ ID NOs. 374/375 |
| HFA20054 | GAG CGG GCG ATC ATC TT | CTC GGC GGC GAT CAC | 33/452 | CG7635 | 18A6 | SEQ ID NOs. 376/377 |
| HFA09675 | GAT GAG AAG GAC GAG AAG AG | CTT GAT GCG GCA ATG GAC | 56/481 | CG8108 | 67C11-D1 | SEQ ID NOs. 378/379 |
| HFA20148 | ATA GGT TCA ACA CGA TCC CC | GAA GGC TGG TGT TAG TTT TG | 93/492 | CG9086 | 1SC5--6 | SEQ ID NOs. 380/381 |
| HFA1 1946 | ACT TGC GTG GAG GAA CTA A | ATG CGT AGA GTT CTT CGG T | 144/490 | Ckllalpha | 80D1 | SEQ ID NOs. 382/383 |
| HFA20230 | AGC TCG AGG ACA ATC CAC | GGC TGA CTT TCA CAG TAG AC | 27/152 | Ckllbeta | 10E3 | SEQ ID NOs. 384/385 |
| HFA09995 | CGT ACG ATG ATG CAC TGG | GAA CGG GCA GAA TGG TTG | 37/499 | comm3 | 71E3--4 | SEQ ID NOs. 386/387 |
| HFA16617 | GGC AGT GGG AGC TCT GA | CTC GGG TCC GGT GAA CT | 30/254 | CtBP | 87D8--9 | SEQ ID NOs. 388/389 |
| HFA19583 | CGT CTG CGC AGT GAT CC | TGG GCT CCG ATG GAT AGA | 39/480 | dome | 18D13--E1 | SEQ ID Nos. 390/391 |
| HFA08714 | AGC GAC GAG GAA GAT GTG | TGA CAA ATG TGG CCT CTG G | 65/476 | dre4 | 6287 | SEQ ID NOs. 392/393 |
| HFA20983 | TTG GAA AAT CGA GAG GAT TTA A | CAC ATT TTT CGA ATT CAA TTG TC | 159/488 | eIF-4B | U | SEQ ID NOs. 394/395 |
| HFA04096 | CGT CTA ATG AGG CAA AGA AAC | CCG TTT TTG CCA CTT TAA CC | 84/487 | enok | 6OB10 | SEQ ID NOs. 396/397 |
| HFA01091 | TCG TGA TGG TGT TGG TGA C | TCC ACT GAA AGT GCT TTG GT | 48/220 | HDC01676 | 30D1 | SEQ ID NOs. 398/399 |
| HFA11427 | GGG CGA ATG CAC GGA AT | TGG CAT ACC TCG AAT AAC TG | 105/477 | HDC11198 | 77D4 | SEQ ID NOs. 400/401 |
| HFA20340 | TAA TCG A CG ATC AGG A AC AG | GTG TGG CCT CGG AGG TG | 52/493 | hop | 10B5--6 | SEQ ID NOs. 402/403 |
| HFA00357 | CGT CCC CCG GTT TTA CG | ATC AGC CAG TCT TGA ATA GTC | 75/488 | lpk2 | 21E2 | SEQ ID NOs. 404/405 |
| HFA04167 | ATA AAA GGC GCC AAG GTG A | TCA CCT GCA TTC CCG TTT C | 18/202 | jbug | 59A3 | SEQ ID NOs. 406/407 |
| HFA07637 | GAC GGG CTT CAA TTC CTA TG | GCG ACG AGG AGA GTG TG | 10/228 | kn | 51C2--3 | SEQ ID NOs. 408/409 |
| HFA19450 | TGC TGC GCA AGC GAC | CAT TTG GCT GGA AGA TGA CA | 38/496 | I(1)G0084 | 18D8--11 | SEQ ID NOs. 410/411 |
| HFA16984 | CAC AAA GCC GCT GAA CAG | TTC GTG GTT ACA CAC ACA GT | 88/496 | larp | 98C3--4 | SEQ ID NOs. 412/413 |
| HFA07247 | CCG CGC GAA CGA CTT | TGA TCG CTT ATC ATC GTA TAT TA | 35/377 | lig | 44A4 | SEQ ID NOs. 414/415 |
| HFA15370 | ACT AGT AGC AGT CAG TCC TC | GCG CCA GCG TTG CTA T | 30/486 | mask | 95F3--5 | SEQ ID NOs. 416/417 |
| HFA20582 | ACA GCA TTC GGG TGG TAA A | GCC ATC CGA AGT TGA TCG | 58/473 | mst | 20A1 | SEQ ID NOs. 418/419 |
| HFA20357 | AAC CAG AAC CAG AAT CAA AAT G | GTT TCC AGC GCG ATT ATT G | 27/118 | nonA | 14B18-C1 | SEQ ID NOs. 420/421 |
| HFA03384 | GCC TGG ATG GAG TTG TTT G | GGA CTT ATG GGC TGA TTG AAC | 87/500 | Nup154 | 32C5 | SEQ ID NOs. 422/423 |
| HFA15220 | AGC GGG TGC AGG AGT TC | TTC TTA TTA CTG GCC ACA TCA T | 36/167 | Obp93a | 93C1 | SEQ ID Nos. 424/425 |
| HFA07660 | CAC GTT CTG CGG TAG CC | GCT TGG GAT CGG CTA AAT C | 60/324 | par-1 | 56D9--11 | SEQ ID NOs. 426/427 |
| HFA16795 | TTG TGG GTA AAT TTT TAC AGA AG | CGA ATT CCC CGC AGT AGT | 12/118 | Pp1alpha-96A | 96A5 | SEQ ID NOs. 428/429 |
| HFA16344 | CGG ATC CGG AGC ACC C | GCG ATG GAG CTG CTG G | 32/469 | PP2A-B' | 90F4--5 | SEQ ID NOs. 430/431 |
| HFA08683 | CTT GAC GCT GAA GAA CCC | CCT GGA ATT GGA TCG ATG C | 72/495 | Ptp61 F | 61F7··62A1 | SEQ ID NOs. 432/433 |
| HFA00777 | GGC AAC CAC TCC ACG CA | TCC TGG CCA GCC GTG T | 28/244 | Rab5 | 22E1 | SEO ID NOs. 434/435 |
| HFA00784 | AGA GCC GCC GAA ACA AC | GGC TTG GTT TCA GTA GAG G | 98/487 | Rrp1 | 23C3··4 | SEQ ID NOs. 436/437 |
| HFA02455 | CAG CAG TAA AGC ACT TTC AA | CCG ATT CCG GCA TGG C | 38/490 | Socs36E | 36E6 | SEQ ID NOs. 438/439 |
| HFA20587 | GAG TAC AAG CAT GTG TAC AAG | GTT CCT GGT GGA GGT AGT G | 31/359 | sol | 19F5 | SEQ ID NOs. 440/441 |
| HFA16870 | CTT GCC CAA AAC TAC AGT TAC | CGA CTG TGG GTG GAT TGT T | 64/479 | Stat92E | 92F1 | SEQ ID NOs. 442/443 |
| HFA11298 | AAG GAA AGC GCA TTT CGT | AAA TCC ATA TCC ACT TCC TCA C | 114/481 | Taf2 | 67D1 | SEQ ID NOs. 444/445 |
| HFA11098 | ATC CCT CAA ATC CCA GTT CC | AAA GTG GCG CTG TGG TG | 53/319 | TSG101 | 73D1 | SEQ ID NOs. 446/447 |

| | | | | | | |
|---|---|---|---|---|---|---|
| A Compete amplicon information can be obtained at http://mai.dkfz.de B Efficiency calculated based on Reynolds et al., (2004). All siRNAs with score of 6 or higher were counted as efficient. · Annotation according to Release 2 of the Berkeley Drosophila Genome Project | | | | | | |

**Table 5: Human homologues of Drosophilagenes with JAK/STAT phenotypes**

| ***Drosophila* Gene** | **BLASTP** | **Identity [%]** | **Human Gene** | **RefSeq protein** | **RefSeq nucleic acid** | **SEQ ID Nos** |
|---|---|---|---|---|---|---|
| | | | | | | |
| Art2 | 1.60E-77 | 44,2 | protein arginine N-methyltransferase 4 | NP_062828.2 | NM 019854 | SEO ID NO. 1/88 |
| asf1 | 3.20E-68 | 61,7 | ASF1 antl-silencing function 1 homolog A | NP_054753.1 | NM 014034 | SEQ ID NO. 2/89 |
| bin3 | 8,80E-49 | 34,3 | hypothetical protein FLJ20257 | NP_062552.2 | NM 019606 | SEQ ID NO. 3/90 |
| bon | 3,60E-45 | 30,5 | tripartite motif-containing 33 protein | NP_56990.2 | NM 015906 | SEQ ID NO. 4/91 |
| Caf1 | 0 | 91,9 | retinoblastoma binding protein 4 | NP_005601.1 | NM 005610 | SEQ ID NO. 5/92 |
| CG10007 | 8,80E-50 | 34,5 | chromosome 2 open reading frame 18 | NP_060347.2 | NM 017877 | SEQ ID NO. 6/93 |
| CG10077 | 7,00E-171 | 67,7 | DEAD (Asp-Glu-Ala-Asp) box polypeptide 5 | NP_004387.1 | NM 004396 | SEO ID NO. 7/94 |
| CG10960 | 3,40E-78 | 36,9 | solute carrier family 2, (facllitated glucose transporter) member 8 | NP_055395.2 | NM 014580 | SEQ ID NO. 8/95 |
| CG11696 | 5,10E-33 | 29,9 | zinc finger protein 502 | NP_149987.2 | NM 033210 | SEQ ID NO. 9/96 |
| CG12460 | 1,80E-17 | 54,0 | splicing factor proline/glutamine rich (polypyrimidine tract binding protein associated) | NP_005057.1 | NM_005066 | SEQ ID NO. 10/97 |
| CG13473 | 3,90E-17 | 34,9 | thioredoxin 2 precursor | NP_036605.2 | NM 012473 | SEQ ID NO. 11/98 |
| CG15306 | 3,30E-27 | 45,1 | microtubule-associated protein, RP/EB family, member 1 | NP_036457.1 | NM 012325 | SEQ ID NO. 12/99 |
| CG15418 | 1,40E-10 | 41,1 | tissue factor pathway inhibitor 2 | NP_006519.1 | NM 006528 | SEQ ID NO. 13/100 |
| CG15434 | 1,60E-17 | 50,6 | NADH dehydrogenase (ubiquinone) 1 alpha subcomplex, 2, 8kDa | NP_002479.1 | NM 002488 | SEQ ID NO. 14/101 |
| CG15706 | 6,60E-20 | 20,0 | FLJ20160 protein | NP_060164.2 | NM 017694 | SEQ ID NO. 15/102 |
| CG16903 | 2,00E-100 | 63,1 | cyclin L1 | NP_064703.1 | NM 020307 | SEQ ID NO. 16/103 |
| CG16975 | 4,00E-123 | 49,4 | I(3)mbt-like 2 isoform a | NP_113676.2 | NM 031488 | SEQ ID NO. 17/104 |
| CG 17492 | 0 | 48,3 | zinc finger, ZZ type with ankyrin repeat domain 1 | NP_543151.1 | NM 080875 | SEQ ID NO. 18/105 |
| CG18112 | 1.80E-20 | 27,8 | chromosome 14 open reading frame 133 | NP_071350.2 | NM 022067 | SEQ ID NO. 19/106 |
| CG30122 | 7,20E-42 | 40,9 | E1B-55kDa-associated protein 5 isoform a | NP_008971.2 | NM 007040 | SEQ ID NO. 20/107 |
| CG3058 | 2.60E-80 | 95,8 | thioredoxin-like 4 | XP_499552.1 | XM 499552 | SEQ ID NO. 21/108 |
| CG31005 | 9.00E·100 | 52,5 | trans-prenyltransferase | NP_055132.2 | NM 014317 | SEQ ID NO. 22/109 |
| CG31132 | 0 | 49,9 | bromo domain-containing protein disrupted in leukemia | NP_694984.2 | NM 153252 | SEQ ID NO. 23/110 |
| CG31358 | 2.10E-51 | 44,2 | stomatln-like 3 | NP_660329.1 | NM 145286 | SEQ ID NO. 24/111 |
| CG31694 | 2,00E-66 | 36,3 | Interferon-related developmental regulator 2 | NP_006755.3 | NM 006764 | SEQ ID NO. 25/112 |
| CG32406 | 5,50E-15 | 37,8 | C1 domain-containing phosphatase and tensin-like protein isoform 3 | NP_938072.1 | NM 198316 | SEQ ID NO. 26/113 |
| CG3281 | 4,00E-41 | 31,7 | zinc finger protein 91 | NP_003421.1 | NM 003430 | SEQ ID NO. 27/114 |
| CG40351 | 2,20E-94 | 56,6 | PREDICTED: KIAA1076 protein | XP_037523.9 | XM 037523 | SEQ ID NO. 28/115 |
| CG4349 | 4,60E-35 | 45,2 | ferritin, heavy polypeptide 1 | NP_002023.2 | NM 002032 | SEQ ID NO. 29/116 |
| CG4446 | 1.90E-66 | 47,2 | pyridoxal kinase | NP_003672.1 | NM 003681 | SEQ ID NO. 30/117 |
| CG4653 | 8,80E-23 | 30,7 | protease, serine, 2 preproprotein | NP_002761.1 | NM 002770 | SEQ ID NO. 31/118 |
| CG4781 | 2,00E-17 | 33,8 | PREDICTED: similar to KIAA0644 protein | XP_379800.1 | XM 379800 | SEQ ID NO. 32/119 |
| CG4907 | 1,10E-47 | 28,9 | phosphatidylinositol glycan, class N | NP_036459.1 | NM 012327 | SEQ ID NO. 33/120 |
| CG6422 | 1,30E-71 | 53,8 | YTH domain family, member 1 | NP_060268.2 | NM 017798 | SEQ ID NO. 34/121 |
| CG6434 | 6,20E-69 | 71,2 | retinoblastoma binding protein 5 | NP_005048.2 | NM 005057 | SEQ ID NO. 35/122 |
| CG6946 | 4.00E-39 | 46,2 | heterogeneous nuclear ribonucleoprotein F | NP_004957 | NM 004966 | SEQ ID NO. 36/123 |
| CG7635 | 2,90E-76 | 62,1 | stomatin isoform a | NP_004090.4 | NM 004099 | SEQ ID NO. 37/124 |
| CG9086 | 0 | 32,0 | ubiquitin protein ligase E3 component n-recognin 1 | NP_777576.1 | NM 174916 | SEQ ID NO. 38/125 |
| Ckllalpha | 4,00E-163 | 88,7 | casein kinase II alpha 1 subunit Isoform a | NP_001886.1 | NM 001895 | SEQ ID NO. 39/126 |
| Ckllbeta | 5.00E-107 | 89,2 | casein kinase 2, beta polypeptide | NP_001311.3 | NM 001320 | SEQ ID NO. 40/127 |
| CtBP | 8.00E-152 | 72,4 | C-terminal binding protein 2 Isoform 1 | NP_001320.1 | NM 001329 | SEQ ID NO. 41/128 |
| dome | 7,60E-15 | 28,2 | sidekick 2 | NP_061937.2 | NM 019064 | SEQ ID NO. 42/129 |
| dre4 | 0 | 59,9 | chromatin-specific transcription elongation factor large subunit | NP_009123.1 | NM 007192 | SEQ ID NO. 43/130 |
| elF-4B | 4,70E-32 | 27,2 | eukaryotic translation Initiation factor 4B | NP_001408.1 | NM 001417 | SEQ ID NO. 44/131 |
| enok | 1,80E-94 | 33,4 | MYST histone acetyltransferase (monocytic leukemia) 3 | NP_006757.1 | NM 006766 | SEQ ID NO. 45/132 |
| HDC01676 | 2,80E-15 | 61,0 | cholinergic receptor, nicotinic, alpha polypeptide 7 precursor | NP_000737.1 | NM 000746 | SEQ ID NO. 46/133 |
| hop | 1,60E-59 | 26,7 | Janus kinase 2 | NP_004963.1 | NM 004972 | SEQ ID NO. 47/134 |
| Ipk2 | 1,80E-26 | 33,6 | Inositol polyphosphate multikinase | NP_689416.1 | NM 152230 | SEQ ID NO. 48/135 |
| jbug | 5,30E-45 | 27,6 | filamin B, beta (actin binding protein 278) | NP_001448.1 | NM 001457 | SEQ ID NO. 49/136 |
| kn | 0 | 69,7 | early B-cell factor | NP_076870.1 | NM 024007 | SEQ ID NO. 50/137 |
| l(1)G0084 | 7,40E-28 | 31,5 | PHD finger protein 10 isoform a | NP_060758.1 | NM 018288 | SEQ ID NO. 51/138 |
| larp | 2,00E-103 | 48,1 | KIAA0731 protein | NP_056130.2 | NM 015315 | SEQ ID NO. 52/139 |
| lig | 5,50E-25 | 32,8 | ubiquitin associated protein 2 isoform 2 | NP_065918.1 | NM 020867 | SEQ ID NO. 53/140 |
| mask | 0 | 74,0 | multiple ankyrin repeats, single KH-domain protein Isoform 1 | NP_060217.1 | NM 017747 | SEQ ID NO. 54/141 |
| mst | 7,00E-52 | 29,7 | misato | NP_060586.2 | NM 018116 | SEQ ID NO. 55/142 |
| nonA | 1,80E-60 | 40,6 | splicing factor proline/glutamine rich (polypyrimidine tract binding protein associated) | NP_005057.1 | NM_005066 | SEQ ID NO. 56/143 |
| Nup154 | 0 | 32,6 | nucleoporin 155kDa isoform 1 | NP_705618.1 | NM 153485 | SEQ ID NO. 57/144 |
| par-1 | 0 | 54,1 | MAP/microtubule affinity-regulating kinase 3 | NP_002367.4 | NM 002376 | SEQ ID NO. 58/145 |
| Pplalpha-96A | 8,00E-169 | 88,9 | protein phosphatase 1, catalytic subunit, alpha isoform 1 | NP_002699.1 | NM_002708 | SEQ ID NO. 59/146 |
| PP2A-B' | 0 | 78,9 | delta isoform of regulatory subunit B56, protein phosphatase 2A isoform 1 | NP_006236.1 | NM_006245 | SEQ ID NO. 60/147 |
| Ptp61F | 5,00E-32 | 37,9 | hypothetical protein LOC9671 | NP_055468 | NM 014653 | SEQ ID NO. 61/148 |
| Rab5 | 4,90E-85 | 75,0 | RABSA, member RAS oncogene family | NP_004153.2 | NM 004162 | SEQ ID NO. 62/149 |
| Rrp1 | 6,10E-82 | 55,2 | APEX nuclease | NP_542380.1 | NM 080649 | SEQ ID NO. 63/150 |
| Socs36E | 4,80E-65 | 68,0 | *suppressor of cytokine signaling 5* | NP_054730.1 | NM 014011 | SEQ ID NO. 64/151 |
| Stat92E | 6,40E-86 | 41,6 | signal transducer and activator of transcription 5B | NP_036580.2 | NM 012448 | SEQ ID NO. 65/152 |
| Taf2 | 0 | 52,5 | TBP-associated factor 2 | NP_003175.1 | NM 003184 | SEQ ID NO. 66/153 |
| TSG101 | 4.30E-98 | 48,7 | tumor susceptibility gene 101 | NP_006283.1 | NM 006292 | SEQ ID NO. 67/154 |

| | | | | | | |
|---|---|---|---|---|---|---|
| Shown are human homologues of *Drosophila* genes with a BLASTP E value of 10⁺¹⁰ or less. | | | | | | |

**Table 6: Human disease homologues of Drosophila genes with JAK/STAT phenotypes**

| ***Drosophlla* Gene** | **SLASTP** | **Human Gene** | **RefSeq protein** | **RefSeq Nucleic acid** | **Disease** | **SEQ ID Nos** |
|---|---|---|---|---|---|---|
| | | | | | | |
| bon | 3.60E-45 | tripartite motif-containing 33 protein | NP_056990.2 | NM 015906 | Thyroid carcinoma, papillary (2) | SEO ID NO. 4/91 |
| Caf1 | 9,90E-17 | peroxin 7 | NP_000279 | NM_000288 | Refsum disease (1) | SEQ ID NO. 68/155 |
| CG10960 | 1,40E-39 | erythrocyte/hepatoma glucose transporter | NP_006507 | NM_006516 | Glucose transport defect, blood-brain barrier (1) | SEQ ID NO. 69/156 |
| CG11696 | 2,60E-25 | zinc finger protein 41 | NP_006051 | NM_006060 | Mental Retardation, X-linked nonsyndromic (1) | SEQ ID NO. 701157 |
| CG17492 | 4,80E-23 | ankyrin, brain | NP_001139 | NM_001148 | Long OT syndrome 4 (1) | SEQ ID NO. 71/158 |
| CG31132 | 2.70E-17 | Lissencephaly-1 Gene | NP_000421 | NM_000430 | Subcortical laminar haterotopia (1) | SEQ ID NO. 72/159 |
| CG31132 | 0 | bromo domain-containing protein disrupted In leukemia | NP_694984 | NM_153252 | Leukemia (3) | SEQ ID NO. 73/160 |
| CG31358 | 7,40E-38 | Podocin | NP_055440 | NM_014625 | Nephrotic syndrome, sleroid-reslstant (1) | SEO ID NO. 74/161 |
| CG32573 | 7,BOE-47 | Protein Kinase C, alpha | NP_002728 | NM_002737 | Pituitary Tumor, invasive (1) | SEO ID NO. 75/162 |
| CG3281 | 2.00E-33 | zinc finger protein 41 | NP_009061 | NM_007130 | Mental Retardation, X-finked nonsyndromic (1) | SEQ ID NO. 76/163 |
| CG40351 | 2.10E-14 | Androgen Receptor·Associated Coregulator 267 | NP_071900 | NM_022455 | Sotos Syndrome, sporadic (1) | SEO ID NO. 77/164 |
| CG4349 | 4,60E-35 | ferritin, heavy polypoplide 1 | NP_002023.2 | NM_002032 | Iron overload, aulosomal dominant (2) | SEO ID NO. 29/116 |
| CG4349 | 3,50E-35 | fth | NP_002023 | NM_002032 | Iron overload, awasomal dominant (1) | SEQ ID NO. 29/116 |
| CG4653 | 6,90E-22 | Protease, Serine, 1 | NP_02760 | NM_002769 | Pancreatitis, hereditary (1) | SEO ID NO. 78/165 |
| CG7635 | 2.90E-76 | stomalin isoform a | NP_004090.4 | NM_004099 | Stomatocytosis (2) | SEO ID NO. 37/124 |
| CG7635 | 5,70E-62 | Podocln | NP_055440 | NM_014625 | Nophrotic syndrome, sterold-reslslant (1) | SEQ ID NO. 74/161 |
| Ckllalpha | 1,20E-23 | serine/threonine protoln kinase 9 | NP_003150 | NM_003159 | Rett Syndrome, atypical (1) | SEQ 10 NO. 78/168 |
| CtBP | 4,20E-24 | 3-phosphogylcerate dehydrogenase; 3pgdh | NP_006614 | NM_006623 | Phosphoglycerate dehydrogenase dellclency (1) | SEQ ID NO. 80/167 |
| dre4 | 4.60E-46 | Lipase A precursor | NP_000226 | NM_000235 | Wolman disease (1) | SEQ ID NO. 81/168 |
| HDC01676 | 2.80E-15 | cholinergic receptor, nicotinic, alpha polypeptide 7 precursor | NP_000737.1 | NM_000746 | Schizophrenia, neurophysiologic defect In (2) | SEQ ID NO. 46/133 |
| hop | 8,40E-52 | Janus kinase 3 | NP_000206 | NM_000215 | SCID, autosomal recessive, T-negative/B-positive type (1) | SEQ ID NO. 82/169 |
| jbug | 5,30E-45 | filamin B, beta (actin binding protein 278) | NP_001448.1 | NM_001457 | Atelostogenesis, type (2) | SEQ tD NO. 49/136 |
| bug | 5,30E-45 | filamin B, beta (actin binding protein 278) | NP_001448.1 | NM_001457 | Larson syndrome (2) | SEQ 10 NO. 49/136 |
| bug | 5,30E-45 | filamin B, beta (actin binding protein 278) | NP_001448.1 | NM_001457 | Spondylocarpotarsal synostosis syndrome (2) | SEO ID NO. 49/136 |
| bug | 6,00E-102 | actin-binding protein 280; abp280 | NP_001447 | NM_001456 | Frontometaphyseal dysplasia (1) | SEO ID NO. 831170 |
| mask | 5,30E-59 | ankyrin, brain | NP_001139 | NM_001148 | Long OT syndrome 4 (1) | SEO ID NO. 71/158 |
| par-1 | 5,30E-39 | Oncogene Akt2 | NP_001617 | NM_001626 | Diabetes mellitus, type II (1) | SEO ID NO. 84/171 |
| Ptp61F F | 7,80E-79 | Protein phosphotyrosylphosphatase 1B | NP_002818 | NM_002827 | Insulin resistance, susceptibility to (1) | SEO ID NO. 85/172 |
| Rab5 | 8,90E-23 | ras-associated protein RAB27A | NP_004571 | NM_004580 | Griseelli Syndrome (1) | SEO ID NO. 86/173 |
| sol | 2,80E-33 | calcium-activated neutral protease 3 | NP_000061 | NM_000070 | Muscular dystrophy, limb-girdle, type 2A (1) | SEO ID NO. 87/174 |
| Stat92E | 6,40E-86 | signal transducer and activator of transcription 5B | NP_036580.2 | NM_012448 | Leukemia, acute promyeloyctlc, STAT5B/RARA type (2) | SEQ ID NO. 65/152 |
| Stat92E | 6,40E-86 | signal transducer and activator of transcription 5B | NP_036580.2 | NM_012448 | Growth hormone Insensitivity with Immunodeficiency (2) | SEO ID NO. 65/152 |
| TSG101 | 4,30E-98 | tumor susceptibility gene 101 | NP_006283.1 | NM_006292 | Breast cancer (2) | SEQ ID NO. 67/154 |
| | | | | | | |

| | | | | | | |
|---|---|---|---|---|---|---|
| Shown are the relevant diseases of the human homologues with a BLASTP E value of 10⁻¹⁰ or less as reterenced In Homophlla (Chien et al., 2002) and OMIM. (1) data sets were downloaded from Homophlla Version 2.1 (update 13 Apr 2005). Chien, S., Reiter, L. T., Bier, E. and Griboskov M., Nucleic Acids Research 30: 149-151 (2002) (2) data sets from: Online Mendelian Inheritance In Man, OMIM (TM). McKusick-Nathans Institute for Genetic Medicine, Johns Hopklns University (Baltimore, MD) and National Center for Biotechnology Information, National Ubrary of Medicine (Bethesda, MD), 2000. World Wide Web URL: http://www.ncbi.nlm.nih.gov/omim/ (3) Information from: Kalla,C., Nentwlch,H., Schlotter,M., Mertens,D., Wildenberger,K., Dohner,H., Stilgenbauer,S. and Lichter,P., Genes Chromosomes Cancer 42 (2): 128-143 (2005) | | | | | | |

**Supplementary Table 7: Expected and Observed Phenotype Frequency**

| **Chromosome** | **No Genes*** | **%** | **Expected Pos** | **Phenotypes Neg** | **Observed Pos** | **Phenotypes Neg** |
|---|---|---|---|---|---|---|
| X | 2292 | 17% | 11 | 4 | 16 | 0 |
| 2L | 2444 | 18% | 11 | 4 | 10 | 5 |
| 2R | 2687 | 20% | 12 | 5 | 5 | 7 |
| 3L | 2612 | 19% | 12 | 4 | 15 | 4 |
| 3R | 3392 | 25% | 16 | 6 | 15 | 8 |
| 4 | 82 | 1% | 1 | 0 | 0 | 0 |
| Unmapped | | | | | 5 | 0 |

| | | | | | | |
|---|---|---|---|---|---|---|
| *Location according to Release 3.1 of the Berkeley Drosophila Genome Project | | | | | | |

## Claims

1. A method for identifying a compound capable of modulating the activity of the JAK/STAT pathway, comprising
(a) contacting a compound with at least one target molecule selected from
(i) nucleic acid molecules, comprising
(i.1) a nucleotide sequence as shown in SEQ ID NOs. 88 to 265;
(i.2) a nucleotide sequence which is complementary to a nucleotide sequence of (i.1);
(i.3) a nucleotide sequence which has an identity of at least 65 % to a nucleotide sequence of (i.1) or (i.2); and/or
(i.4) a nucleotide sequence which hybridizes under stringent conditions to a nucleotide sequence of (i.1), (i.2) or (i.3);
and
(ii) polypeptide molecules
(ii.1) encoded by the nucleic acid molecules of (i) and/or
(ii.2) having the sequences as shown in SEQ ID NOs. 1-87,
and
(b) determining the degree of modulation of the at least one target molecule by the compound.

2. The method according to claim 1, wherein the compound is selected from compounds capable of directly and/or indirectly inhibiting or activating the transcription or translation of a nucleic acid molecule of (i).

3. The method according to claim 2, wherein the compounds capable of directly and/or indirectly inhibiting or activating the transcription or translation of a nucleic acid molecule of (i) comprise polypeptides such as proteins, enzymes, antibodies, polypeptide inhibitors, polypeptide activators, agonist, antagonists, mimetics, low molecular weight substances, antisense molecules, RNAi molecules and ribozymes.

4. The method according to claim 1, wherein the compound is selected from compounds capable of directly and/or indirectly inhibiting or activating a polypeptide molecule of (ii).

5. The method according to claim 4, wherein the compounds capable of directly and/or indirectly inhibiting or activating a polypeptide molecule of (ii) comprise polypeptides such as proteins, enzymes, antibodies, polypeptide inhibitors, polypeptide activators, agonist, antagonists, mimetics, oligopeptides, low molecular weight substances and cofactors.

6. The method according to any one of claims 1 to 5, wherein the compound is an antibody or fragment thereof and wherein the antibody or fragment thereof is directed against a polypeptide molecule of (ii).

7. The method according to any one of claims 1 to 5, wherein the compound is an antisense molecule and wherein the antisense molecule is directed against a nucleic acid molecule of (i).

8. The method according to any one of claims 1 to 5, wherein the compound is an RNAi molecule.

9. The method according to any one of claims 1 to 8, wherein the degree of modulation of the at least one target molecule by the compound is determined by measuring the amount and/or expression rate of the nucleic acid molecule of (i).

10. The method according to any one of claims 1 to 8, wherein the degree of modulation of the at least one target molecule by the compound is determined by measuring the amount and/or activity of the polypeptide molecule of (ii).

11. The method according to any one of claims 1 to 10, wherein the method is a molecular based assay.

12. The method according to any one of claims 1 to 10, wherein the method is a cellular assay.

13. Use of at least one molecule selected from
(i) nucleic acid molecules, comprising
(i.1) a nucleotide sequence as shown in SEQ ID NOs. 88 to 265;
(i.2) a nucleotide sequence which is complementary to a nucleotide sequence of (i.1);
(i.3) a nucleotide sequence which has an identity of at least 65 % to a nucleotide sequence of (i.1) or (i.2); and/or
(i.4) a nucleotide sequence which hybridizes under stringent conditions to a nucleotide sequence of (i.1), (i.2) or (i.3);
and
(ii) polypeptide molecules
(ii.1) encoded by the nucleic acid molecules of (i) and/or
(ii.2) having the sequences as shown in SEQ ID NOs. 1-87,
as a target for the modulation of the activity of the JAK/STAT pathway.

14. A method for modulating the activity of the JAK/STAT pathway comprising contacting a cell with at least one molecule selected from
(i) nucleic acid molecules, comprising
(i.1) a nucleotide sequence as shown in SEQ ID NOs. 88 to 265;
(i.2) a nucleotide sequence which is complementary to a nucleotide sequence of (i.1);
(i.3) a nucleotide sequence which has an identity of at least 65 % to a nucleotide sequence of (i.1) or (i.2); and/or
(i.4) a nucleotide sequence which hybridizes under stringent conditions to a nucleotide sequence of (i.1), (i.2) or (i.3);
(ii) polypeptide molecules
(ii.1) encoded by the nucleic acid molecules of (i) and/or
(ii.2) having the sequences as shown in SEQ ID NOs. 1-87, and
(iii) effector molecules of (i) and/or (ii).

15. The method according to claim 14, wherein the effector molecules of (i) and/or (ii) are selected from antibodies or fragments thereof which are directed against a polypeptide molecule of (ii), antisense molecules which are directed against a nucleic acid molecule of (i) and RNAi molecules.

16. A pharmaceutical composition comprising as an active agent at least one molecule selected from
(i) nucleic acid molecules, comprising
(i.1) a nucleotide sequence as shown in SEQ ID NOs. 88 to 265;
(i.2) a nucleotide sequence which is complementary to a nucleotide sequence of (i.1);
(i.3) a nucleotide sequence which has an identity of at least 65 % to a nucleotide sequence of (i.1) or (i.2); and/or
(i.4) a nucleotide sequence which hybridizes under stringent conditions to a nucleotide sequence of (i.1), (i.2) or (i.3);
(ii) polypeptide molecules
(ii.1) encoded by the nucleic acid molecules of (i) and/or
(ii.2) having the sequences as shown in SEQ ID NOs. 1-87,
and
(iii) effector molecules of (i) and/or (ii).

17. The pharmaceutical composition according to claim 16, wherein the effector molecules of (i) and/or (ii) are selected from antibodies or fragments thereof which are directed against a polypeptide molecule of (ii), antisense molecules which are directed against a nucleic acid molecule of (i) and RNAi molecules.

18. The pharmaceutical composition according to claim 16 or 17, optionally containing pharmaceutically acceptable carriers, diluents and/or adjuvants.

19. the pharmaceutical composition according to any one of claims 16 to 18 for the diagnosis, prevention or treatment of a JAK/STAT pathway associated disorder.

20. The pharmaceutical composition according to any one of claims 16 to 19, wherein the JAK/STAT pathway associated disorder is selected from the group consisting of papillary thyroid carcinoma, Refsum disease, blood-brain barrier glucose transport defect, X-linked nonsyndromic mental retardation, long QT syndrome 4, subcortical laminar heterotopia, leukemia, steroid-resistant nephrotic syndrome, invasive pituitary tumor, sporadic Sotos syndrome, autosomal dominant iron overload, hereditary pancreatitis, stomatocytosis I, atypical Rett syndrome, phosphoglycerate dehydrogenase deficiency, Wolman disease, neurophysiologic defect in schizophrenia, autosomal recessive SCID (T-negative/B-positive type), atelostogenesis (type I), Larson syndrome, spondylocarpotarsal synostosis syndrome, frontometaphyseal dysplasia, diabetes mellitus (type II), susceptibility to insulin resistance, Griscelli Syndrome, limb-girdle muscular dystrophy (type 2A), growth hormone insensitivity with immunodeficiency and breast cancer.

21. Use of at least one molecule selected from
(i) nucleic acid molecules, comprising
(i.1) a nucleotide sequence as shown in SEQ ID NOs. 88 to 265;
(i.2) a nucleotide sequence which is complementary to a nucleotide sequence of (i.1);
(i.3) a nucleotide sequence which has an identity of at least 65 % to a nucleotide sequence of (i.1) or (i.2); and/or
(i.4) a nucleotide sequence which hybridizes under stringent conditions to a nucleotide sequence of (i.1), (i.2) or (i.3);
(ii) polypeptide molecules
(ii.1) encoded by the nucleic acid molecules of (i) and/or
(ii.2) having the sequences as shown in SEQ ID NOs. 1-87,
and
(iii) effector molecules of (i) and/or (ii);
for the manufacture of a pharmaceutical composition for the diagnosis, prevention or treatment of a JAK/STAT pathway associated disorder.

22. Use according to claim 21, wherein the effector molecules of (i) and/or (ii) are selected from antibodies or fragments thereof which are directed against a polypeptide molecule of (ii), antisense molecules which are directed against a nucleic acid molecule of (i) and RNAi molecules.

23. Use according to claim 21 or 22, wherein the JAK/STAT pathway associated disorder is selected from the group consisting of papillary thyroid carcinoma, Refsum disease, blood-brain barrier glucose transport defect, X-linked nonsyndromic mental retardation, long QT syndrome 4, subcortical laminar heterotopia, leukemia, steroid-resistant nephrotic syndrome, invasive pituitary tumor, sporadic Sotos syndrome, autosomal dominant iron overload, hereditary pancreatitis, stomatocytosis I, atypical Rett syndrome, phosphoglycerate dehydrogenase deficiency, Wolman disease, neurophysiologic defect in schizophrenia, autosomal recessive SCID (T-negative/B-positive type), atelostogenesis (type I), Larson syndrome, spondylocarpotarsal synostosis syndrome, frontometaphyseal dysplasia, diabetes mellitus (type II), susceptibility to insulin resistance, Griscelli Syndrome, limb-girdle muscular dystrophy (type 2A), growth hormone insensitivity with immunodeficiency and breast cancer.
